# EUROPEAN PATENT APPLICATION

(11) **EP 1 672 074 A1**
(43) Date of publication of application: **21.06.2006**
(21) Application number: 05112867.6
(22) Date of filing: 23.05.2001
(51) Int. Cl.: C12N 15/54, C12N 9/10, C12N 1/21, C12P 19/18, C08B 37/00, C12R 1/225

(54) **Lactobacillus reuteri glucosyltransferase**

(30) Priority: 25.05.2000 EP 00201871
(62) Divisional of application: 01934631.1
(71) Applicant: Nederlandse Organisatie voor Toegepast-Natuurwetenschappelijk Onderzoek TNO, 2628 VK Delft (NL)
(72) Inventor: van Geel-Schutten, Gerritdina Hendrika, 3971 XG Driebergen (NL); Dijkhuizen, Lubbert, 9472 RB Zuidlaren (NL); Rahaoui, Hakim, 3813 VS Amersfoort (NL); Leer, Robert Jan, 3902 PN Veenendaal (NL)
(74) Representative: van Westenbrugge, Andries

(57) **Abstract**

The present invention describes a protein having glucosyltransferase activity. This protein is derived from lactobacilli, which are food-grade microorganisms with the Generally Recognised As Safe (GRAS) status. The protein produces a glucan with a unique structure having 4-linked, 6-linked and 4,6-linked anhydroglucose units or in the presence of suitable acceptors, oligosaccharides. According to the invention lactobacilli capable of producing this glucan using the novel glucosyltransferase can be used as a probiotic or symbiotic.

## Description

The present invention is in the field of enzymatic production of biomolecules. The invention is particularly concerned with a novel type of glucosyltransferase derived from lactobacilli and with a process for recombinant production of the enzyme and for the production of useful glucans and gluco-oligosaccharides from sucrose.

### Background of the invention

Lactic acid bacteria (LAB) play an important role in the fermentative production of food and feed. Traditionally, these bacteria have been used for the production of for instance wine, beer, bread, cheese and yoghurt, and for the preservation of food and feed, e.g. olives, pickles, sausages, sauerkraut and silage. Because of these traditional applications, lactic acid bacteria are food-grade micro-organisms that posses the Generally Recognised As Safe (GRAS) status. Due to the different products which are formed during fermentation with lactic acid bacteria, these bacteria contribute positively to the taste, smell and preservation of the fmal product. The group of lactic acid bacteria includes several genera such as *Lactobacillus, Leuconostoc, Pediococcus, Streptococcus,* etc.

In recent years also the health promoting properties of lactic acid bacteria have received much attention. They produce an abundant variety of exopolysaccharides (EPS's). These polysaccharides are thought to contribute to human health by acting as prebiotic substrates, nutraceuticals, cholesterol lowering agents or immunomodulants.

To date high molecular weight polysaccharides produced by plants (such as cellulose, starch and pectin), seaweeds (such as alginate and carrageenan) and bacteria (such as alginate, gellan and xanthan) are used in several industrial applications as viscosifying, stabilising, emulsifying, gelling or water binding agents. Although all these polysaccharides are used as food additives, they originate from organisms not having the GRAS status. Thus they are less desirable than the exopolysaccharides of micro-organisms, such as lactic acid bacteria, which have the GRAS status.

The exopolysaccharides produced by lactic acid bacteria can be divided in two groups, heteropolysaccharides and homopolysaccharides; these are synthesised by totally different mechanisms. The former consist of repeating units in which residues of different types of sugars are present and the latter consist of one type of monosaccharide. The synthesis of heteropolysaccharides by lactic acid bacteria, including lactobacilli, has been studied extensively in recent years. Considerable less information is present on the synthesis of homopolysaccharides from lactobacilli, although some studies have been performed. The information on the synthesis of homopolysaccharides in lactobacilli is mainly limited to the synthesis of glucans and only two reports, written by the present inventors, exist on the synthesis of fructans. In one of these reports the *Lactobacillus reuteri* strain LB 121 was found to produce both a glucan and a fructan when grown on sucrose, but only a fructan when grown on raffinose (van Geel-Schutten, G.H. *et al.,* Appl. Microbiol. Biotechnol. (1998) 50, 697-703). In the other report was found that *Lactobacillus reuteri* strain LB 35-5, a spontaneous mutant of *Lactobacillus reuteri* strain LB 121, only produced a glucan when grown on sucrose (van Geel-Schutten, G.H. *et al.,* Appl. Environ. Microbiol. (1999) 65, 3008-3014). In the other report the soluble glucan and fructan were also characterised by their molecular weights (of 3,500 and 150 kDa respectively) and the glucan was reported to be highly branched with a unique structure consisting of a terminal, 4-substituted, 6-substituted, and 4,6-di-substituted α-glucose in a molar ratio 1.1 : 2.7 : 1.5 : 1.0 (van Geel-Schutten, G.H. *et al.,* Appl. Environ. Microbiol. (1999) 65, 3008-3014). No structurally identification of a similar glucan produced by a *Lactobacillus* had been reported before. The fructan was identified as a (2→6)-β-D-fructofuranan (also called a levan). This was the first example of levan synthesis by a *Lactobacillus* species.

### Summary of the invention

A novel enzyme having glucosyltransferase activity using sucrose as a substrate has now been found in *Lactobacillus reuteri,* and its amino acid sequence and other structural properties have been determined. The enzyme is unique in that it is capable of producing a highly branched glucan with α-1,4- and α-1,6-glucosidic links. The invention thus pertains to an enzyme, to DNA encoding it, to cells containing such DNA and to their use in producing carbohydrates, as defined in the appending claims. The invention also pertains to glucans so produced as well as to oligosaccharides and chemically derivatised glucans.

### Description of the invention

It was found according to the invention that the glucans are produced by certain *Lactobacillus* strains, in particular by certain strains of *Lactobacillus reuteri,* as a result of the activity of a single glucosyltransferase (glucansucrase).

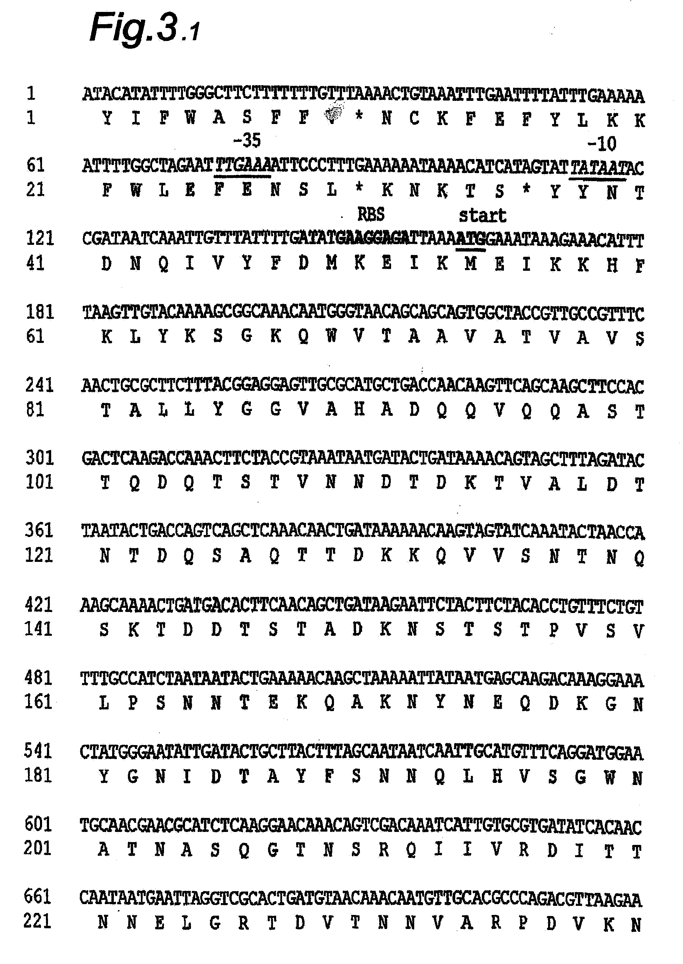

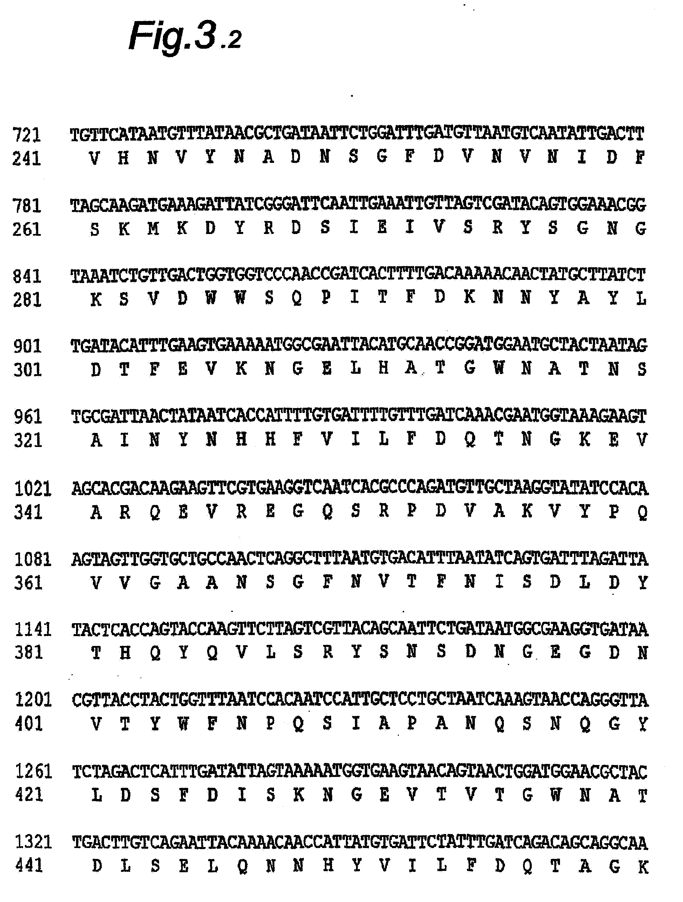

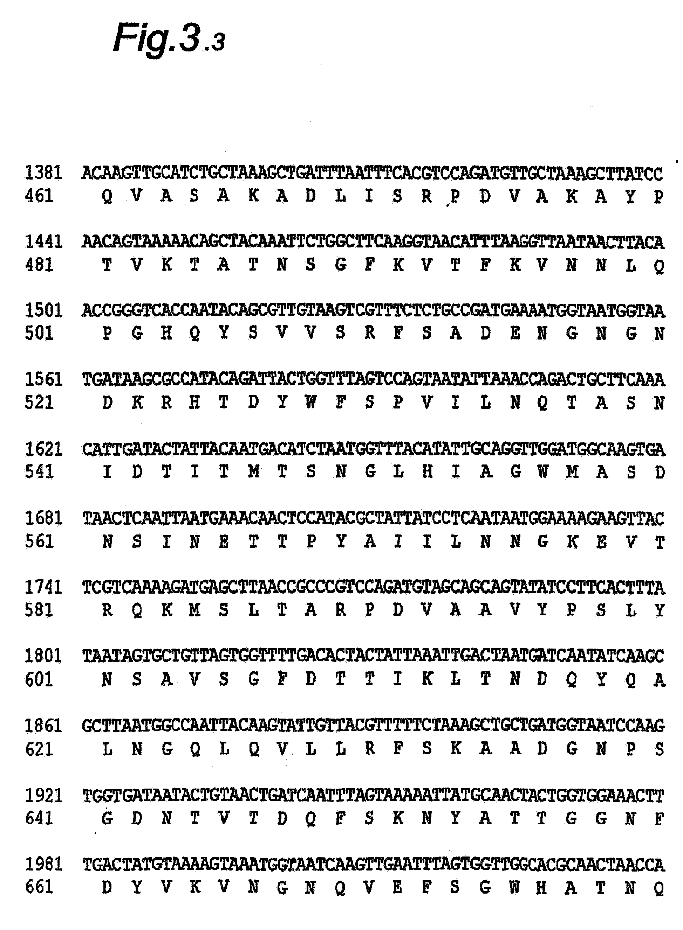

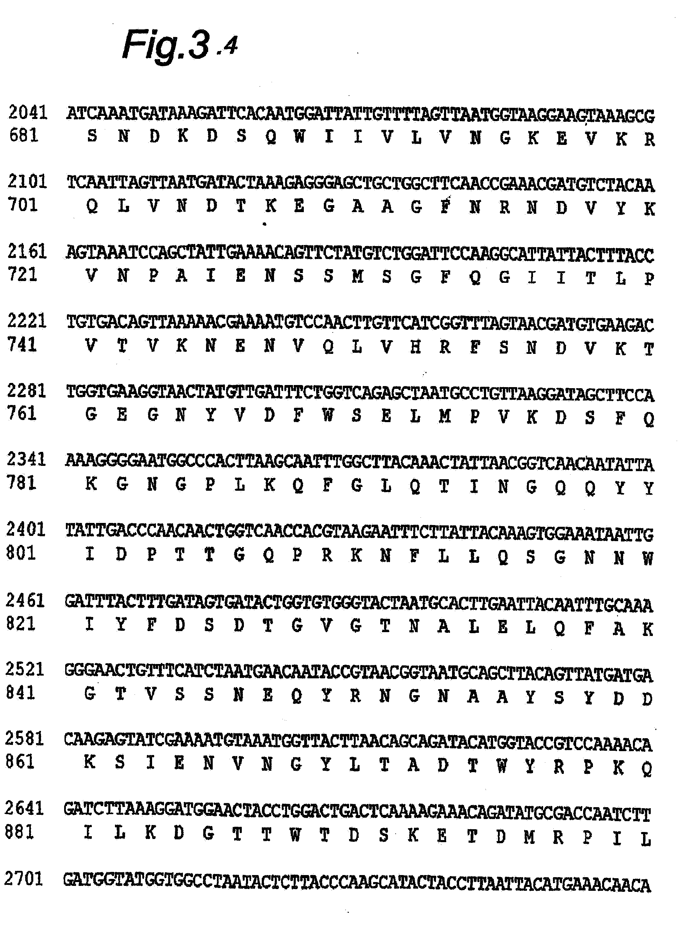

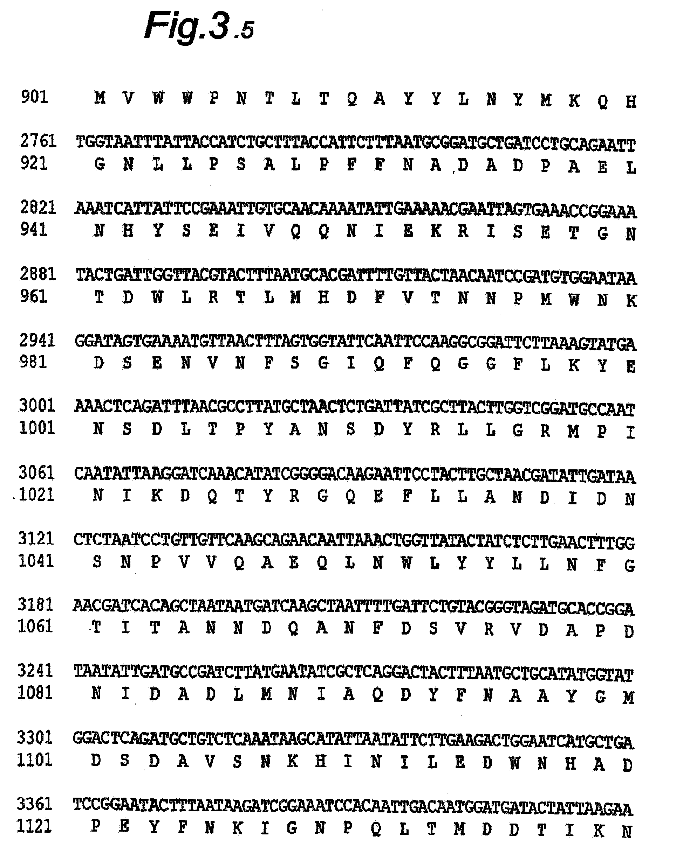

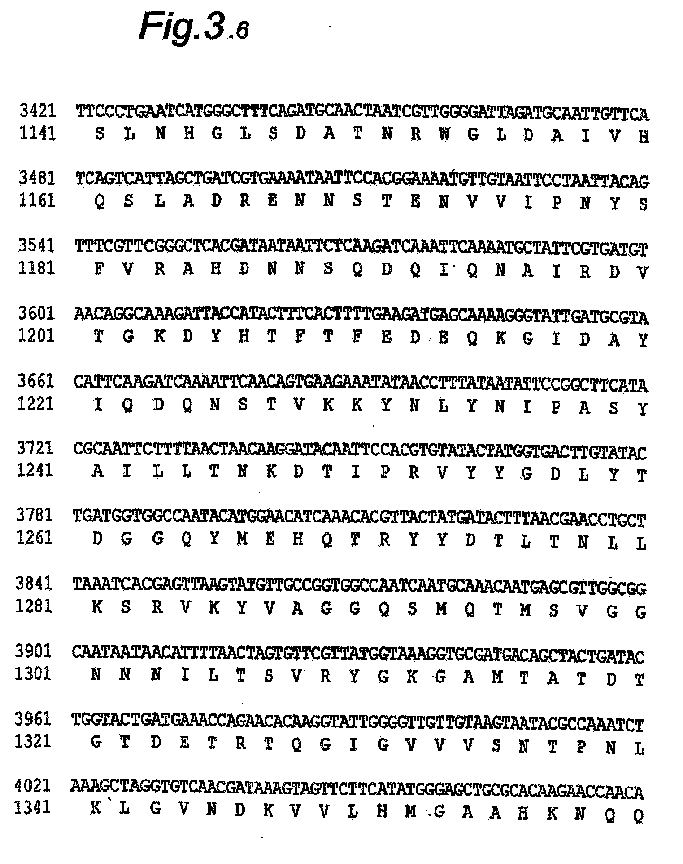

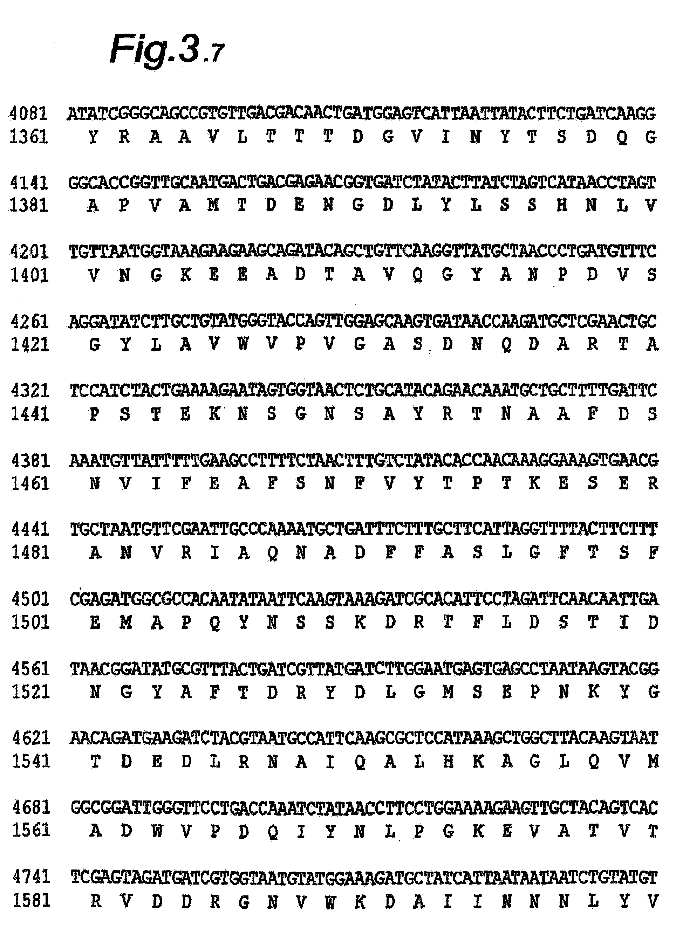

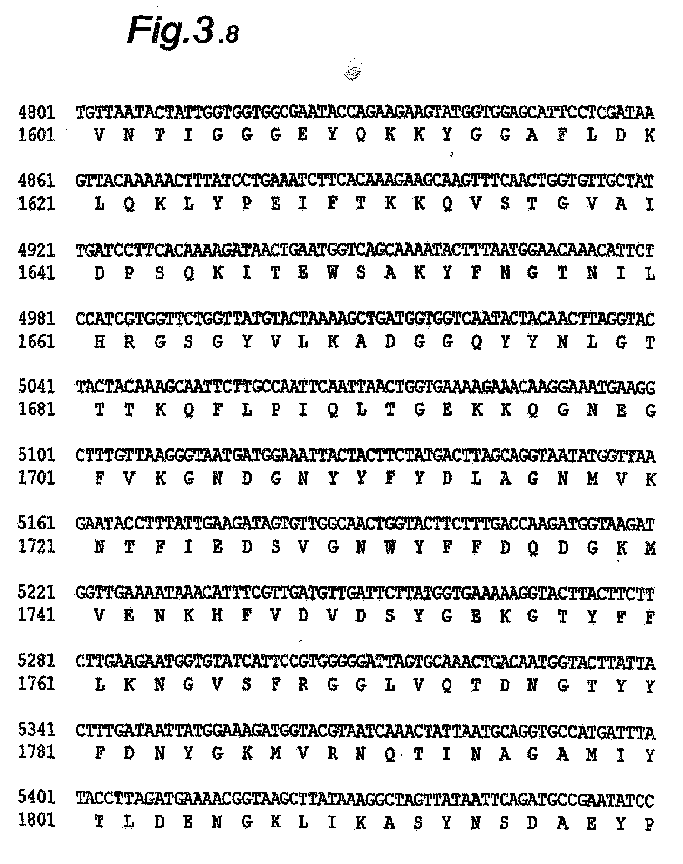

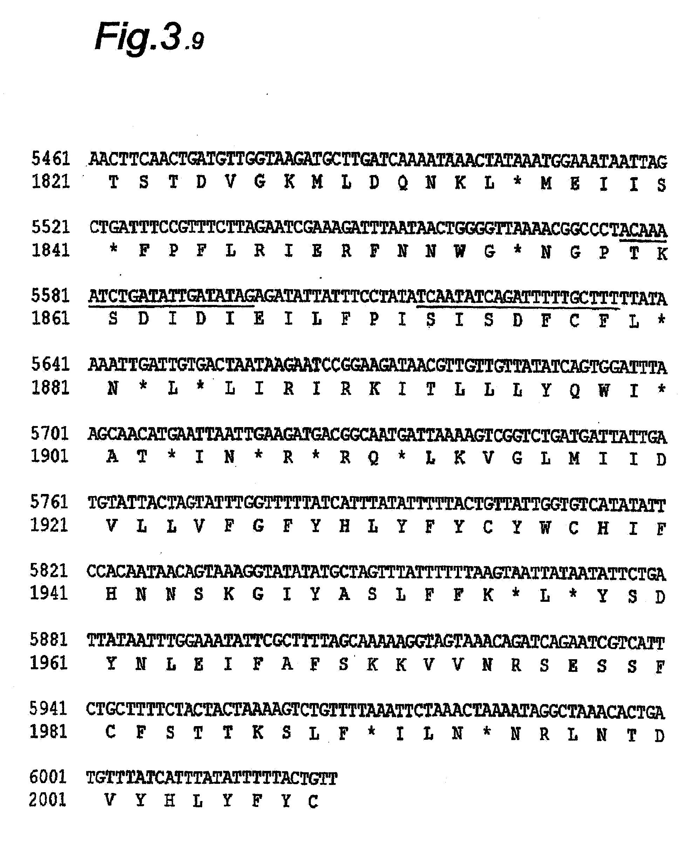

The nucleotide and amino acid sequence of the novel glucosyltransferase are represented by SEQ ID No. 1 and 2, respectively, and are also shown in figure 3. The start codon of the glucosyltransferase is preceded by a putative ribosome binding site with the nucleotide sequence GAAGGAGA (located 5 base pairs upstream of the start codon of the glucosyltransferase; see SEQ ID No. 1 and figure 3). Furthermore, the start codon is preceded by the nucleotide sequence TATAAT, also called Pribnow box or -10 region, (located 42 base pairs upstream of the start codon; see SEQ ID No. 1 and figure 3) and by the nucleotide sequence TTGAAA, also called -35 region (located 80 base pairs upstream of the start codon; see SEQ ID No. 1 and figure 3).

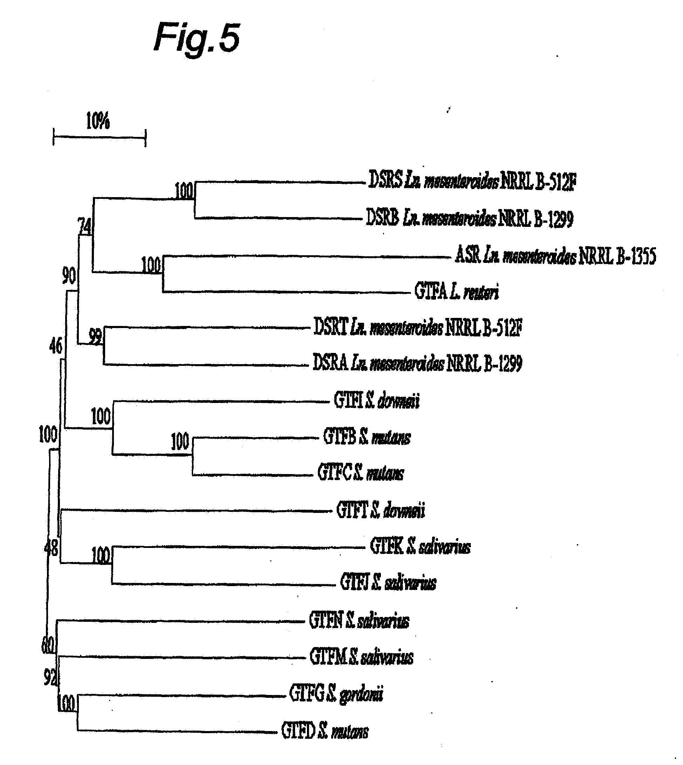

The invention covers a protein having glucosyltransferase activity with sucrose as substrate comprising an amino acid sequence with an amino acid identity of at least 50%, preferably at least 60%, and more preferably at least 70%, compared to the amino acid sequence 531-1781 of SEQ ID No. 2. The invention also covers a part of a protein with at least 15 contiguous amino acids which are identical to the corresponding part of the amino acid sequence 531-1781 of SEQ ID No. 2. The present invention covers a protein having glucosyltransferase activity with sucrose as donor substrate with an amino acid identity of at least 50%, preferably at least 60%, and more preferably at least 70%, compared to the amino acid sequence of SEQ ID No. 2. The invention also covers a part of a protein with at least 15 contiguous amino acids which are identical to the corresponding part of the amino acid sequence of SEQ ID No. 2. The novel glucosyltransferase has homology with several other proteins as revealed by amino acid sequence alignment. A high homology (figure 5) was found with an alternansucrase of *Leuconostoc mesenteroides* strain NRRL B-1355 (46% identity, within 1261 amino acids of amino acid sequence 531-1781 of SEQ ID No. 2) and a dextransucrase of *Leuconostoc mesenteroides* strain NRRL B-512F (44% identity, within 1270 amino acids of amino acid sequence 531-1781 of SEQ ID No. 2). Furthermore, the alignment revealed the presence of various domains also found in the other glucosyltransferases, such as an N-terminal variable domain, a catalytic domain and a C-terminal glucan binding domain. The N-terminal domain shows almost no identity with the N-terminal domains of other glucosyltransferases and an N-terminal signal peptide could not be detected.

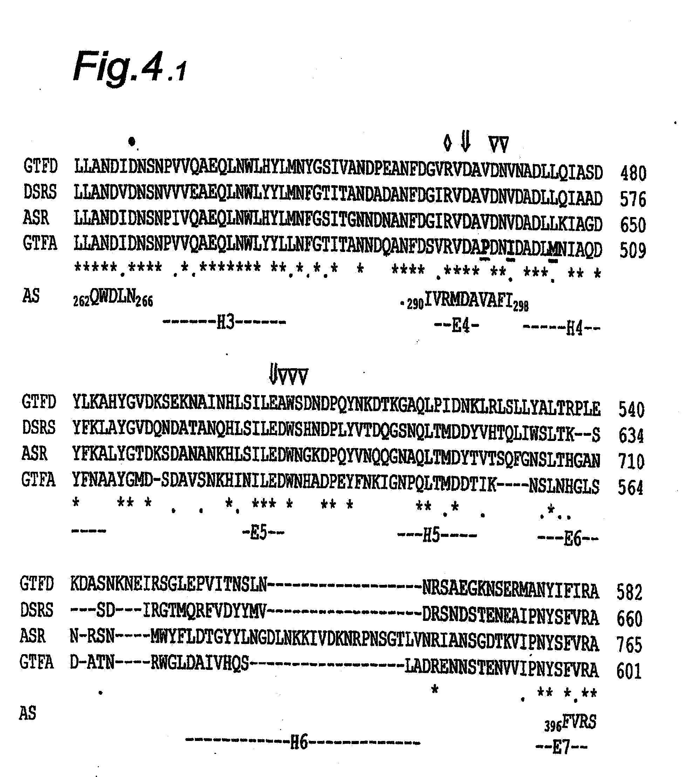

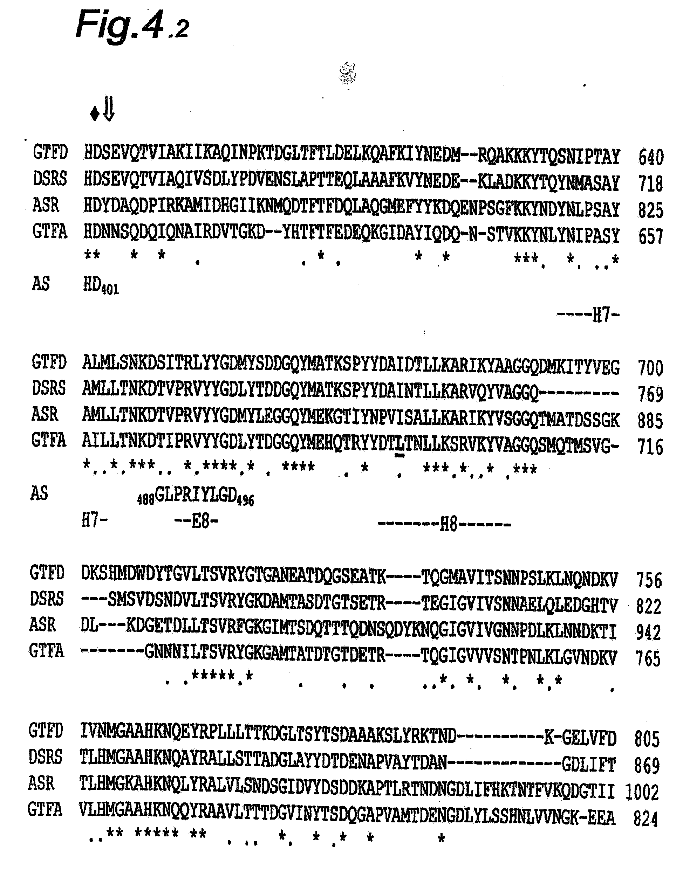

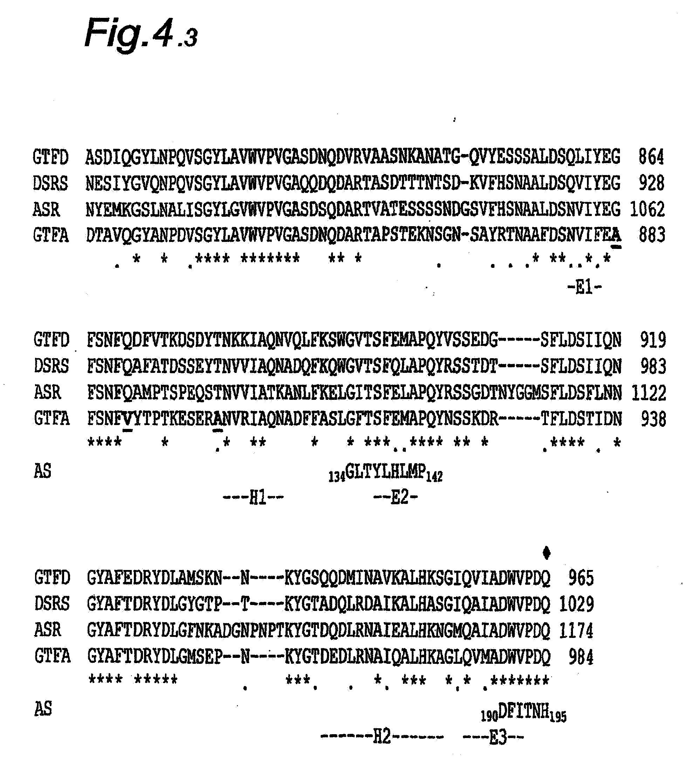

The invention also covers a protein comprising an amino acid sequence of at least 100 amino acids, exhibiting at least 55%, preferably at least 65% amino acid identity with the corresponding part of the amino acid sequence 972-1514 (catalytic domain) of SEQ ID No. 2. The catalytic domain shows a high level of homology (about 50% identity) with other known streptococcal and *Leuconostoc* glucosyltransferases and putative functions based on the alignment can be ascribed to several amino acids within this catalytic domain (figure 4). Asp-1024, Glu-1061 and Asp-1133 of SEQ ID No. 2 are putative catalytic residues, Asp-984 of SEQ ID No. 2 is a putative calcium binding residue and Arg-1022 of SEQ ID No. 2 a putative chloride binding residue. His-1132 and Gln-1514 of SEQ ID No. 2 may stabilise the transition state and the residues Asp-1027, Asn-1028, Asp-1062 and Trp-1063 of SEQ ID No. 2 may play a role in binding of acceptor molecules and in the transfer of the glucosyl moiety.

The invention further covers a protein comprising an amino acid sequence of at least 100 amino acids, exhibiting at least 50%, preferably at least 60%, amino acid identity with the corresponding part of the amino acid sequence 1515-1781 (glucan binding domain) of SEQ ID No. 2. The C-terminal putative glucan binding domain is much shorter than the corresponding domains in other glucosyltransferases but three known repeats, resembling YG repeats, are described: YYFYDLAGNMVKN (SEQ ID No. 3) starting at amino acid position 1656 of SEQ ID No. 2, WYFFDQDGKMVEN (SEQ ID No. 4) starting at amino acid position 1678 of SEQ ID No. 2 and TYYFDNYGKMVRN (SEQ ID No. 5) starting at amino acid position 1725 of SEQ ID No. 2. YG repeats are defined by the presence of one or more aromatic residues (of which one is usually tyrosine), followed by 3-4 glycine residues downstream, a hydrophobic residue, a neutral polar residue (usually glycine or asparagine) and 1-3 hydrophobic residues. It is striking that the number of repeats necessary to ensure glucan binding properties is different for enzymes producing a soluble or an insoluble glucan. Possibly the glucan binding domain is also involved in the determination of the glucan structure and the polymer chain growth. Furthermore, this domain seems also necessary for the complete glucosyltransferase activity.

Specific amino acids of the glucosyltransferase that are believed to be important for the unique properties of the enzyme include Pro-1026, Ile-1029, Met-1034, Asn-1035, Ser-1136, Ala-1143, Ile-1170, Leu-1223, Ala-1413, Val-1418, Ala-1428, Leu-1442 of the amino acid sequence of SEQ ID No. 2. So a protein, mutant or part thereof, comprising at least one of the above mentioned amino acids is also part of the invention. Particularly Pro-1026 and Ile-1029 are of interest. Pro-1026 is found in a position where a conserved Val is found in other glucosyltransferases. Compared to Val, the presence of Pro results in a more rigid protein structure. This change of protein structure may influence the glucosidic bonds formed and might explain the unique structure of the glucan. Ile-1029 is also found in a position where a conserved Val is present in other LAB glucosyltransferases not producing α(1,4) bonds. An identical amino acid substitution is observed in amylosucrase, a glucosyltransferase synthesising α(1,4) bonds.

A nucleotide sequence encoding any of the above mentioned proteins, mutants, variants or parts thereof is also a subject of the invention. Furthermore, the nucleic acid sequences corresponding to expression-regulating regions (promoters, enhancers, terminators) contained in the nucleic acid sequence (1)-(160) or (5507)-(6026) of SEQ ID No. 1 (see also figure 3) can be used for homologous or heterologous expression of genes. Such expression-regulating sequences are operationally linked to a polypeptide-encoding nucleic acid sequence such as the genes of the glucosyltransferase according to the invention. Inverted repeats are located 62 base pairs downstream the termination codon (TAA), suggesting the presence of a Rho independent transcription termination signal. The -10 and -35 consensus promoter sequences, two motifs generally present upstream of the start codon of procaryotes, are identified as described above. Other promoter, enhancer or terminator were not identified. A nucleic acid construct comprising the nucleotide sequence operationally linked to an expression-regulating nucleic acid sequence is also covered by the invention.

A recombinant host cell, such as a mammalian (with the exception of human), plant, animal, fungal or bacterial cell, containing one or more copies of the nucleic acid construct mentioned above is an additional subject of the invention. A part of the gene encoding the N-terminal part, the catalytic domain and the glucan-binding domain of the glucosyltransferase has been cloned and expressed in *E*. *coli.* The molecular weight predicted from the deduced amino acid sequence of that part *(i.e.* the amino acid sequence 531-1781 of SEQ ID No. 2; see examples) of the recombinant glucansucrase expressed in *E. coli* is 145 kDa.

The invention further covers a enzymatically active protein as defined above which, in the presence of sucrose, produces a glucan having 38-48% 4-linked anhydroglucose units, 17-28% 6-linked anhydroglucose units, and 7-20% 4,6-linked anhydroglucose units, preferably a glucan having 40-46% 4-linked anhydroglucose units, 19-26% 6-linked anhydroglucose units, and 9-18% 4,6-linked anhydroglucose units. There is a large variation in glucans due to differences in the type of bonds present, degree and type of branching, length of the glucan chains, molecular weight, and the conformation of the polymers. The structure of this glucan is unique in that it is highly branched, consists of terminal, 4-substituted, 6-substituted, and 4,6-di-substituted α-glucose in a molar ratio 1.1 : 2.7 : 1.5 : 1.0 and has a high molecular weight of 3500 kDa. The novel glucan may be synthesised by a glucosyltransferase present in the *Lactobacillus* strains, preferably *Lactobacillus reuteri* strains and more preferably *Lactobacillus reuteri* strains LB 121 and LB 35-5. *Lactobacillus reuteri* belongs to the group of lactic acid bacteria which are known to play an important role in the fermentative production of food and feed. Because of this, lactic acid bacteria are food-grade micro-organisms that posses the Generally Recognised As Safe (GRAS) status.

The invention also pertains to a process of producing a glucan as described above using an isolated glucosyltransferase according to the invention and a suitable glucose source such as for instance sucrose. The glucosyltransferase may be isolated by conventional means from the culture of a glucosyltransferase-positive lactic acid bacterium, especially a *Lactobacillus reuteri,* or from a recombinant organism expressing the glucosyltransferase gene.

Additionally, the invention concerns a process of producing gluco-oligosaccharides containing the characteristic structure of the glucan described above using an isolated glucosyltransferase according to the invention. There is a growing interest in oligosaccharides derived from homopolysaccharides, for instance for prebiotic purposes. Several fructo- and gluco-oligosaccharides are known to stimulate the growth of bifidobacteria in the human colon. Gluco-oligosaccharides produced by the glucosyltransferase described above can be used as prebiotics and probiotics and are also part of the invention. The production of the gluco-oligosaccharides is different from the glucan synthesis reaction. In addition to sucrose, the substrate of the glucosyltransferase, an acceptor molecule such as maltose or lactose is necessary for the acceptor reaction. Another way of producing gluco-oligosaccharides is by hydrolysis of the glucan described above. This hydrolysis can be performed by known hydrolysis methods such as enzymatic hydrolysis with enzymes such as amylase, dextranase or pullulanase or by acid hydrolysis. The produced gluco-oligosaccharides must contain at least one 1,6- or one 4,6- glucosidic link to be used as prebiotics.

The invention also concerns chemically modified glucans and gluco-oligosaccharides based on the 1,4/1,6-α-glucans described above. Chemical modification can be achieved by oxidation, such as hypochlorite oxidation resulting in ring-opened 2,3-dicarboxy-anhydroglucose units (see e.g. EP-A-427349), periodate oxidation resulting in ring-opened 2,3-dialdehyde-anhydroglucose units (see e.g. WO 95/12619), which can be further oxidised to (partly) carboxylated units (see e.g. WO 00/26257), TEMPO-mediated oxidation resulting in 6-carboxy-anhydroglucose units (see e.g. WO 95/07303). The oxidised glucans have improved water-solubility, altered viscosity and a retarded fermentability and can be used as metal-complexing agents, detergent additives, strengthening additives, bioactive carbohydrates, emulsifiers and water binding agents. They can also be used as starting materials for further derivatisation such as cross-linking and the introduction of hydrophobes. Oxidised glucans coupled to proteins can be used as emulsifiers and stabilisers. (Partial) hydrolysis of glucans according to the invention and/or modified glucans results in gluco-oligosaccharides, which can be used as bioactive carbohydrates or prebiotics. The oxidised glucans of the invention preferably contain 0.05-1.0 carboxyl groups per anhydroglucose unit, e.g. as 6-carboxyl units.

Another type of chemical modification is phosphorylation, as described in O.B. Wurzburg (1986), Modified Starches: properties and uses. CRC Press Inc., Boca Raton, 97-112. One way to achieve this modification is by dry heating glucans with a mixture of monosodium and disodium hydrogen phosphate or with tripolyphosphate. The phosphorylated glucans are suitable as wet-end additives in papermaking, as binders in paper coating compositions, as warp sizing-agents, and as core binders for sand molds for metal casting. A further type of derivatisation of the glucans is acylation, especially acetylation using acetic or propionic anhydride, resulting in products suitable as bleaching assistants and for the use in foils. Acylation with e.g. alkenyl succinic anhydrides or (activated) fatty acids results in surface-active products suitable as e.g. surfactants, emulsifiers, and stabilisers.

Hydroxyalkylation, carboxymethylation, and amino-alkylation are other methods of chemical derivatisation of the glucans. Hydroxyalkylation is commonly performed by base-catalysed reaction with alkylene oxides, such as ethylene oxide, propylene oxide or epichlorohydrine; the hydroxyalkylated products have improved solubility and viscosity characteristics. Carboxymethylation is achieved by reaction of the glucans with monochloroacetic acid or its alkali metal salts and results in anionic polymers suitable for various purposes including crystallisation inhibitors, and metal complexants. Amino-alkylation can be achieved by reaction of the glucans with alkylene-imines, halo-alkyl amines or amino-alkylene oxides, or by reaction of epichlorohydrine adducts of the glucans with suitable amines. These products can be used as cationic polymers in a variety of applications, especially as a wet-end additive in paper making to increase strength, for filler and fines retention, and to improve the drainage rate of paper pulp. Other potential applications include textile sizing and wastewater purification. The above mentioned modifications can be used either separately or in combination depending on the desired product. Furthermore, the degree of chemical modification is variable and depends on the intended use. If necessary 100% modification, i.e. modification of all anhydroglucose units can be performed. However, partial modification, e.g. from 1 modified anhydroglucose unit per 100 up to higher levels, will often be sufficient in order to obtain the desired effect.

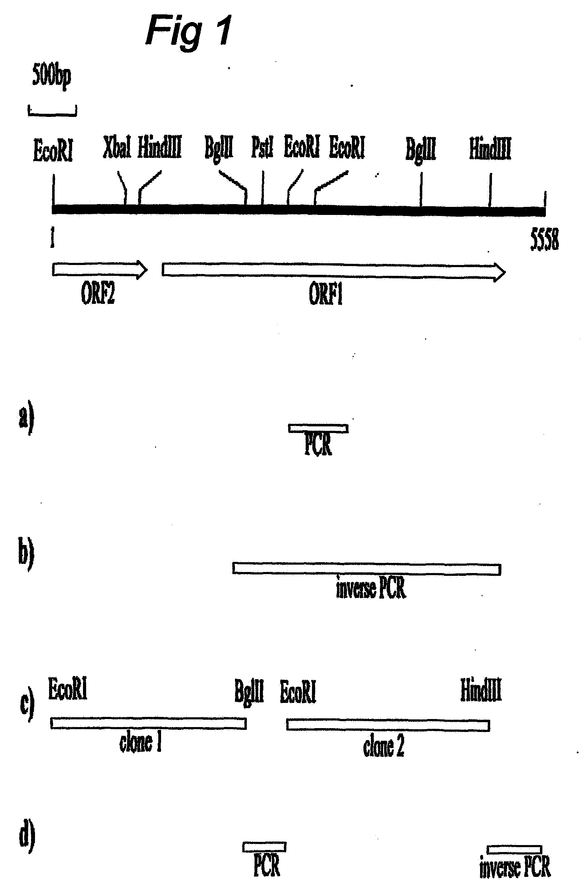

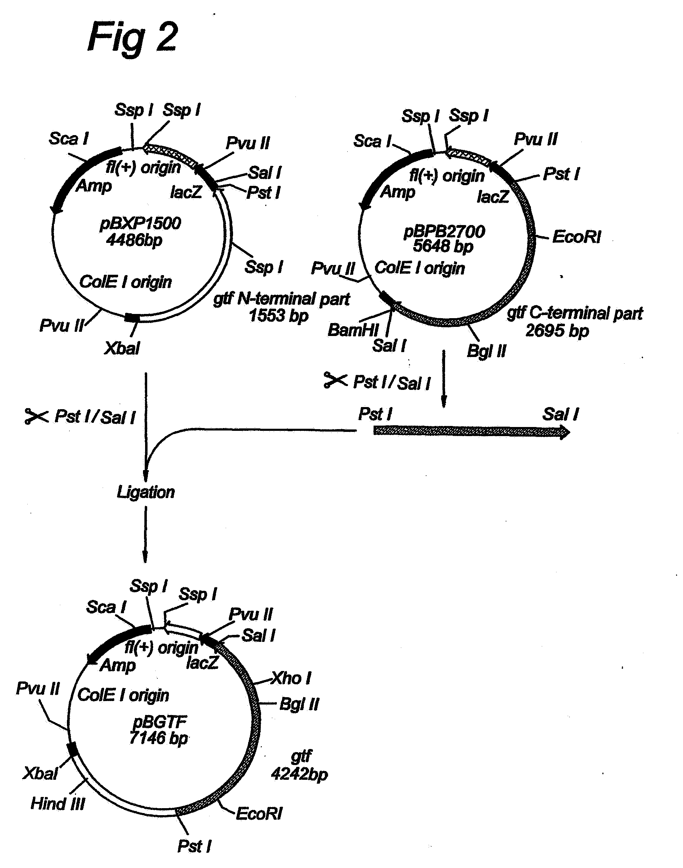

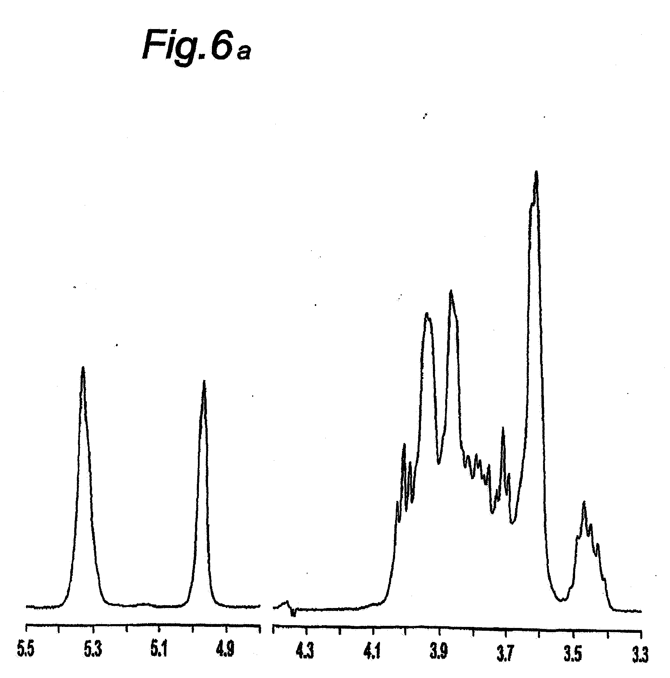

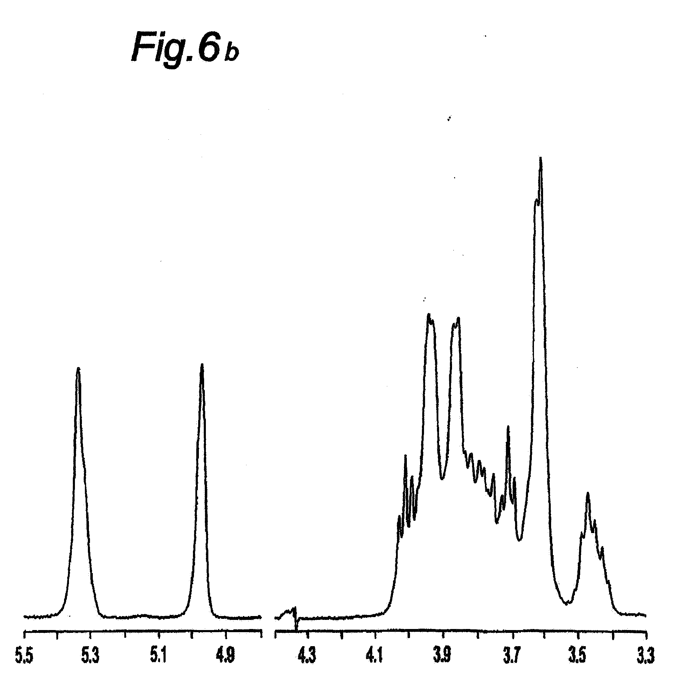

The use of a *Lactobacillus* strain capable of producing the novel and unique glucan is also covered by the invention. Preferably, the strain is also capable of producing a fructan, which can be either a levan, inulin or both. More preferably, the strain is also capable of producing fructo-oligosaccharides. For producing this glucan and/or fructan the strain according to the invention preferably uses sucrose as a substrate. The glucan according to the invention can be produced when the strain is cultured in the presence of sucrose and/or after the strain was cultured in the presence of sucrose during a sufficient time period. The *Lactobacillus* strains used have been deposited at the BCCM/LMG Culture collection (Gent, BE) under the deposit numbers LMG P-18388 *(L. reuteri* wild-type strain LB 121) and LMG P-18389 *(L. reuteri* mutant strain LB 35-5). The efficacy of some *L. reuteri* strains as a probiotic has been demonstrated in various animals such as for instance poultry and humans. The administration of *L. reuteri* to pigs resulted in significantly lower serum total and LDL-cholesterol levels, while in children *L. reuteri* is used as a therapeutic agent against acute diarrhea. For this and other reasons *L. reuteri* has already been supplemented to commercially available probiotic products. The mode of action of *L. reuteri* as a probiotic is still unclear. Preliminary studies indicated that gut colonisation by *L. reuteri* may be of importance. According to the invention, it was found that the mode of action of *L. reuteri* as a probiotic may reside partly in the ability to produce polysaccharides. *Lactobacillus* strains, preferably *L. reuteri* strains, more preferably *L. reuteri* strains LB 121, LB 35-5 and other strains capable of producing a glucan having 38-48% 4-linked anhydroglucose units (AGU), 17-28% 6-linked AGU, and 7-20% 4,6-linked AGU, preferably a glucan having 40-46% 4-linked AGU, 19-26% 6-linked AGU, and 9-18% 4,6-linked AGU can thus advantageously be used as a probiotic. They can also, together with these polysaccharides, be used as a symbiotic.
***Examples***
Example 1: Isolation of DNA from *Lactobacillus reuteri,* nucleotide sequence analysis of the glucosyltransferase gene, construction of plasmids for expression of the glucosyltransferase gene in *E. coli* DH5α, expression of an essential part of the glucosyltransferase gene in *E. coli* DH5α, and identification of the novel glucan produced by the recombinant enzyme.
General procedures for cloning, DNA manipulations and agarose gel electrophoresis were essentially as described by Sambrook *et al.* (1989) Molecular cloning: a laboratory manual, 2nd ed. Cold Spring Harbour Laboratory Press, Cold Spring Harbour, New York. Restriction endonuclease digestions and ligations with T4 DNA ligase were performed as recommended by the suppliers. DNA was amplified by PCR techniques using ampliTAQ DNA polymerase (Perkin Elmer). DNA fragments were isolated from agarose gels using the Qiagen extraction kit (Qiagen GMBH), following the instructions of the suppliers. *Lactobacillus reuteri* strains were grown anaerobically at 37°C in MRS medium (DIFCO) or in MRS-s medium (MRS medium containing 100 g/l sucrose instead of 20 g/l glucose) and *E. coli* strains were grown aerobically at 37°C in LB medium containing 100 µg/ml ampicillin (when appropriate 40 µg/ml X-gal was added).
For the isolation of chromosomal DNA, *L. reuteri* 121 was grown overnight at 37°C in MRS broth (Difco) supplemented with 40 mM DL-threonine. Cells of 4 ml culture were harvested by centrifugation and resuspended in 10 ml MRS broth supplemented with 40 mM DL-threonine and incubated for 2 h at 37°C. After centrifugation the cells were resuspended in 400 µl protoplast buffer (10 mM sodium maleate, pH 6.1 supplemented with 0.3 M lactose, 10 mM MgCl₂, 12% polyethyleneglycol 2000, 0.1 M EDTA, 5 mg/ml lysozyme (47,000 U/mg) and 10 U/ml mutanolysine) and incubated for 1 h at 37°C. After centrifugation (1 min, Eppendorf centrifuge), protoplasts were resuspended in 500 µl 20 mM Tris-HCl, pH 8,0. Subsequently, 100 µl laurylsarcosine and 150 µl 5 M NaCl were added and DNA was extracted. Plasmid DNA of *Lactobacillus reuteri* was isolated using a modification of the methods of Anderson and Mc Kay (1983) Appl. Environ. Microbiol. 46, 549-552 and Burger and Dicks (1994) Biotechnol. Technol. 8, 769-772. Fresh prewarmed (37°C) MRS broth (10 ml) was inoculated with 200 µl of an overnight culture and incubated for 2.5 h at 37°C. Cells were harvested by centrifugation and washed with 2 ml sterile STE buffer (0.1 M NaCl, 10 mM Tris-HCl, 1 mM EDTA, pH 8). After centrifugation, the pellet was resuspended in 380 µl solution I (0.5 M sucrose, 50 mM Tris-HCl, 1 mM EDTA, pH 8, containing 2 mg/ml lysozyme and 6.6 U mutanolysin). After an incubation of 1.5 h at 37°C, 50 µl of solution II (50 mM Tris-HCl, pH 80, 0,25 M EDTA) and 30 µl of solution III (50 mM Tris-HCL, pH 8, 20 mM EDTA, 20% SDS) were added and the suspension was mixed. Sodium hydroxide (30 µl of a 3 M solution) was added, followed by 50 µl 2 M Tris-HCl and 72 µl 5 N NaCl. After extraction with equal volumes of phenol and chloroform, the DNA was precipitated with ethanol.
The glucosyltransferase (*gtfA*) gene was identified by amplification with PCR using degenerated primers (GTFpr1, 5'GAYAAKWSNAAKSYNRTNGTNSARGC3' (SEQ ID No. 6) and GTFpr2, 5'GNKCNCANATRATRCCNCTRNA3' (SEQ ID No. 7); Y=T or C, K=G or T, W=A or T, S=C or G, R=A or G, N= A, C, G, or T) based on conserved amino acid sequences deduced from different glucosyltranferase genes (*gtfS* of *Streptococcus downei, gtfC* of S. *mutans, gtfI* of S. *downei, gtfK* and *gtfM* of S. *salivarius* and *dsrA* of *Leuconostoc mesenteroides)* and *Lactobacillus reuteri* chromosomal DNA as template. An amplification product with the predicted size of about 660 bp was obtained (figure 1A). To investigate the possible presence of multiple copies of the glucosyltransferase gene, Southern hybridisation was performed. DNA was restricted with endonucleases, separated by agarose gel electrophoresis and transferred to a Hybond nylon membrane. For hybridisation, probes were labelled wih [α-³²P]dCTP using Random Primed DNA labelling kit (Boehringer Mannheim), following the manufacturer's instructions. The Southern hybridisation of chromosomal DNA of the *Lactobacillus reuteri* strain 121 with the amplified 660 bp PCR fragment, followed by washing under non-stringent conditions (45°C, 0.5x SSC/0.1 SDS) revealed one hybridising fragment, suggesting the presence of only a single copy of a glucosyltransferase gene in the *L*. *reuteri* strains. The 660 bp fragment was cloned in *E. coli* JM109 using the pCR2.1 vector. Transformations were performed by electroporation using the BioRad gene pulser apparatus at 2.5 kV, 25 µF and 200 Ω, following the instructions of the manufacturer. The fragment was sequenced by the method of Sanger *et al.* (1977) Proc. Natl. Acad. Sci. USA 74, 5463-5467, confirming that the correct part of the *gtfA* gene had been isolated. The 660 bp amplified fragment was used to design primers for inverse PCR. Using inverse PCR techniques a 3 kb fragment of the isolated *gtfA* gene was generated (figure 1B). This 3 kb amplicon was identified by sequencing and probes were designed to isolate the *Eco*RI/*Bgl*II and *Eco*RI/*Hind*III fragments from a partial DNA library of *L. reuteri* in *E*. *coli* DH5α (figure 1C). Positive clones were selected by colony blot hybridisation using Hybond-N filters, following the instructions of the supplier and the cloned fragments were sequenced. Attempts to clone the C-terminal part of the glucansucrase gene in *E. coli* DH5α or JM109 using a partial DNA library strategy with different vectors failed. Therefore, the C-terminal part was isolated by inverse PCR. The remaining fragment, located between the *Eco*RI/*Bgl*II and *Eco*RI/*Hind*III fragments, was isolated by PCR techniques (figure 1D). The amplicons obtained were sequenced directly. To eliminate errors due to the PCR reaction, these fragments were sequenced for at least 4 times, using different clones per PCR reaction. Both DNA strands of the entire glucosyltransferase gene were sequenced twice. In this way the sequence of a 5.5 kb region of the *Lactobacillus reuteri* chromosomal DNA, containing the *gtfA* gene and its surroundings, were obtained.
The plasmids for expression of the glucosyltransferase gene in *E. coli* DH5α were constructed as described hereafter. A 4.8 kb fragment, containing the entire glucosyltransferase gene was generated by PCR, using the primers GTFpr3 (5'ACAACCACCA TGGAATTAGG TCGCACTGAT GTAAC3') (SEQ ID No. 8) and GTFpr4 (5'GCCAGCTGGA TCCGTCGACT AGTTTATTTT TGATCAAGCA TCTTACC3') (SEQ ID No. 9). Both primers contained suitable restriction enzyme recognition sites at their 5' ends *(Nco*I in GTFpr3 and *Bam*HI and *Sal*I in GTFpr4). Cloning of this PCR fragment in different vectors failed. Therefore, the strategy depicted in figure 2 was followed. Briefly, the PCR product was restricted with *Xba*I/*Pst*I and *Pst*I/*Bam*HI (figure *1; Bam*HI site was introduced with GTFpr4). The resulting fragments (1503 bp and 2696 bp, respectively) were cloned separately in pBluescriptIISK⁺ yielding pBXP1500 and pBPB2700. Ligation of the 2700 bp *Pst*I/*Sal*I fragment isolated from pBPB2700 in pBXP1500, digested with *Pst*I and *Sal*I*,* yielded pBGTF (7146 bp) in *E. coli* DH5α. Plasmid DNA of *E. coli* was isolated using the alkaline lysis method of Birnboim and Doly (1979) Nucleic. Acid Res. 7, 1513-1523 or with a Qiagen plamid kit following the instructions of the supplier. Cells of *E. coli* DH5α with pBGTF were harvested by centrifugation after 16h of growth. The pellet was washed with 50 mM sodium acetate buffer pH 5.5 containing 1 mM CaCl₂ and 1% (v/v) Tween-80 and the suspension was centrifuged again. Pelleted cells were resuspended in 50 mM sodium acetate buffer pH 5.5 containing 1 mM CaCl₂, 1% (v/v) Tween-80 and 7.2 mM β-mercaptoethanol. Cells were broken by sonication. Cell debris and intact cells were removed by centrifugation for 15 min at 4°C at 14,000 rpm in an Eppendorf centrifuge and the resulting cell free extract was used in the enzyme assays.
The glucosyltransferase activity was determined at 37°C by monitoring the release of fructose from sucrose or by measuring the amount of glucan produced using *E. coli* cell free extracts or *Lactobacillus reuteri* culture supernatant in reaction buffer (50 mM sodium acetate, 1 mM CaCl₂, 1% (v/v) Tween-80, 10 g/l sucrose, pH 8). Sucrose, glucose and fructose were determined using commercially available kits. For determination of the molecular weight of the glucosyltransferase produced by *E*. *coli* or *L. reuteri,* SDS-PAGE was performed according to Laemmli (1970) Nature 227, 680-685. SDS-PAGE gels were stained using the PAS activity staining. Glucans were collected by precipitation with ethanol. ¹H-NMR spectroscopy (figure 6) and methylation analysis (table 1) were performed as described by van Geel-Schutten *et al.* (1999) Appl. Environ. Microbiol. 65, 3008-3014. The molecular weights of the glucans were determined by high performance size exclusion chromotography coupled on-line with a multi angle laser light scattering and a differential refractive index detector. After the first nucleotide sequencing of the obtained DNA two putative start codons leading to either a protein encoded by 3834 nucleotides (starting at nucleotide position 1670 of SEQ ID No. 1) or a protein encoded by 3753 nucleotides (starting at nucleotide position 1751 of SEQ ID No. 1) were identified. Both putative start codons were preceded by a putative ribosome binding site, GCAGG (located 4 base pairs upstream of nucleotide position 1670 of SEQ ID No. 1) or AGAAG (located 14 base pairs upstream of nucleotide position 1751 of SEQ ID No. 1), respectively. Depending on the potential start codon used, one of these glucosyltransferases comprised 1278 amino acids (starting at amino acid positions 504 of SEQ ID No. 2) (3834 nucleotides) and the other comprised 1251 amino acids (starting at amino acid position 531 of SEQ ID No. 2) (3753 nucleotides). The molecular weight (MW) deduced of the amino acid sequence of these glucosyltransferases was 143 and 140 kDa, respectively. The isoelectric point deduced of the amino acid sequence of these glucosyltranferases was 4.73 (for the higher MW protein) and 4.71 (for the lower MW protein), at pH 7, respectively. Surprisingly, the molecular weight of the purified protein from *Lactobacillus reuteri* indicated by SDS-PAGE was not approximately 140 kDa, but about 190 kDa. After repeating the nucleotide sequencing, it appeared that the above mentioned nucleotide forms did not represent the complete nucleotide sequence of the glucosyltransferase according to the invention, but were merely a part of the complete nucleotide sequence encoding the protein of the invention. The complete nucleotide sequence of the novel glucosyltransferase is represented in SEQ ID No. 1 and the amino acid sequence of said glucosyltransferase is shown in SEQ ID No. 2. All experiments were performed with both the complete nucleotide or amino acid sequence of the protein (SEQ ID No. 1 and 2, respectively) and the partial nucleotide or amino acid sequence mentioned above. The results of the experiments performed with the complete or partial amino acid sequence and the complete or partial nucleotide sequence mentioned above were identical indicating that the part of the glucosyltransferase represented by said partial nucleotide and amino acid sequences is essential for the functionality of the glucosyltransferase according to the invention.

**Table 1: Methylation analysis of the glucans produced by Lactobacillus reuteri strains and E. coli GTFA.**

| Type of glucosyl units | *Lactobacillus reuteri* strain 121 | *Lactobacillus reuteri* strain 35-5 | *E. coli* GTFA |
|---|---|---|---|
| Glc*p*-(1 → | 24% | 25% | 21% |
| →4)-Glc*p*-(1→ | 42% | 43% | 44% |
| →6)-Glc*p*-(1→ | 22% | 21% | 24% |
| →4,6)-Glc*p*-(1→ | 12% | 11% | 11% |

Example 2: Adhesion experiments with *Lactobacillus* strains.
The adhesion of *Lactobacillus reuteri* strains to Caco-2 cell lines was determined as described below. Firstly, a bacterial suspension was prepared as follows. *L. reuteri* strains LB 121, 35-5 and *L. rhamnosus* LGG (a well known probiotic strain with good adhering properties) were cultured in MRS broth supplemented with 5 µl/ml of methyl-1,2-[³H]-thymidine at 37°C for 18-20 h before the adhesion assays. The cultures were harvested by centrifugation, washed with phosphate buffered saline (PBS) and resuspended in PBS or PBS supplemented with 30 g/l sucrose (see Table 2) to a final density of about 2 x 10⁹ cfu/ml. Prior to the adhesion assay, the cell suspensions in PBS with 30 g/l sucrose were incubated for 1 hour at 37°C, whereas the cell suspensions in PBS were kept on ice for 1 hour. After incubation at 37°C, the suspensions in PBS with sucrose were centrifuged and the cells were washed with and resuspended in PBS to a final density of about 2 x 10⁹ cfu/ml.
Caco-2 cells were cultured as follows. Subcultures of Caco-2 cells (ATCC, code HTB 37, human colon adenocarcinoma), stored as frozen stock cultures in liquid nitrogen were used for the adhesion tests. The Caco-2 cells were grown in culture medium consisting of Dulbecco's modified Eagle medium (DMEM), supplemented with heat-inactivated foetal calf serum (10% v/v), non-essential amino acids (1% v/v), L-glutamine (2mM) and gentamicin (50 µg/ml). About 2,000,000 cells were seeded in 75cm² tissue culture flasks containing culture medium and cultured in a humidified incubator at 37°C in air containing 5% CO₂. Near confluent Caco-2 cell cultures were harvested by trypsinisation and resuspended in culture medium. The number of cells was established using a Bürker-Türk counting chamber.

**Table 2: Incubation of the different Lactobacillus strains prior to the adhesion assays.**

| *Lactobacillus* strain | Extra incubation | Polysaccharide produced | Group |
|---|---|---|---|
| *reuteri* 121 | PBS sucrose, 37°C, 1 hr | glucan and fructan | As |
| *reuteri* 35-5 | PBS sucrose, 37°C, 1 hr | glucan | Bs |
| *reuteri* K24 | PBS sucrose, 37°C, 1 hr | none | Cs |
| *reuteri* 121 | PBS on ice | none | D |
| *reuteri* DSM20016* | PBS on ice | none | E |
| *rhamnosus* GG | PBS on ice | none | F |

| | | | |
|---|---|---|---|
| *Type strain of *L. reuteri* | | | |

For the following experiments a Caco-2 monolayer transport system was used. Caco-2 cells cultured in a two-compartment transport system are commonly used to study the intestinal, epithelial permeability. In this system the Caco-2 cell differentiates into polarised columnar cells after reaching confluency. The Caco-2 system has been shown to simulate the passive and active transcellular tranport of electrolytes, sugars, amino acids and lipophilic compounds (K.M. Hillgren, A, Kato, R.T. Brochardt, Medical Research Reviews 15: 83-109, 1995; W.J. Dulfer, J.P. Groten, H.A.J. Govers, J. Lipid Res. 37: 950-961, 1996; E. Duizer, A.H. Penninks, W.H. Stenhuis and J.P. Groten, J. Contr. Rel. 49: 39- 49, 1997). Also, a clear correlation between the *in vivo* absorption and the permeability across the monolayers of Caco-2 cells has been reported (P. Artursson and J. Karlsson, Biochem. Biophys. Res. Commun. 75: 880-885, 1991). For the present transport studies, Caco-2 cells were seeded on semi-permeable filter inserts (12 wells Transwell plates, Costar) at ca. 100,000 cells per filter (growth area ± 1 cm² containing 2.5 ml culture medium). The cells on the insert were cultured for 17 to 24 days at 37°C in a humidified incubator containing 5% CO₂ in air. During this culture period the cells have been subjected to an enterocyte-like differentiation. Gentamycin was eliminated from the culture medium two days prior to the adhesion assays.
The adhesion assay was performed as follows. PBS was used as exposure medium. 25 µl of a bacterial suspension (2×10⁹ cfu/ml) were added to 0.5 ml medium. The apical side of the Caco-2 monolayers was incubated with the bacterial suspensions for 1 hour at 37°C. After incubation, remaining fluid was removed and the cells were washed three times with 1 ml PBS. Subsequently, the Caco-2 monolayers were digested overnight with 1 ml 0.1M NaOH, 1% SDS. The lysate was mixed with 10 ml Hionic Fluor scintillation liquid and the radioactivity was measured by liquid scintillation counting using a LKB/-Wallac scintillation counter. As a control, the radioactivity of the bacterial suspensions was measured. For each test group, the percentage of bacteria attached to the monolayers was calculated. All adhesion tests were performed in quadruple. In Table 3 the results of the bacterial adhesion test to Caco-2 cell lines are given. From the results can be concluded that the glucans and the fructans contribute to the adherence of *L. reuteri* to Caco-2 cell lines. This could indicate that *L. reuteri* strains producing EPS possess improved probiotic characteristics or that *L. reuteri* and its polysaccharides could function as an excellent symbiotic.

**Table 3: The results of the bacterial adhesion test to Caco-2 cell lines.**

| Group (see Table 1) | % of bacteria bound to the monolayer |
|---|---|
| As | 6.5 |
| Bs | 5.7 |
| Cs | 1.8 |
| D | 2.3 |
| E | 0.9 |
| F | 1.3 |

Example 3: Oxidation of glucans.
For TEMPO-mediated oxidation, a glucan produced as described above (dry weight 1 g, 6.15 mmol) was resuspended in 100 ml water. Next, 2,2,6,6-tetramethylpiperidine-1-oxyl (TEMPO; 1% by weight compared to the polysaccharide (0.01 g, 0.065 mmol)) was added and resuspended in 20 min. Sodium bromide (0.75 g, 7.3 mmol) was added and the suspension was cooled to 0°C. The reaction can also be performed without bromide. A solution of hypochlorite (6 ml, 15% solution, 12.6 mmol) was adjusted to pH 10.0 with 3M HCl and cooled down to 0°C. This solution was added to the suspension of the polysaccharide and TEMPO. The course of the reaction was followed by monitoring the consumption of sodium hydroxide solution, which is equivalent to the formation of uronic acid. After 30 min, the amount of 0.1M NaOH consumed corresponded to the formation of 96% uronic acid, with respect to the available units having a primary hydroxyl group. Thereafter, the solution was poured out in 96% ethanol (comprising 70% of the volume of the solution) causing the product to precipitate. The white precipitate was centrifuged, resuspended in ethanol/water (70/30 v/v) and centrifuged again. Next, the precipitate was resuspended in 96% ethanol and centrifuged. The obtained product was dried at reduced pressure. The uronic acid content was determined by means of the uronic acid assay according to Blumenkrantz and Abdoe-Hansen (Anal. Biochem., 54 (1973), 484). A calibration curve was generated using polygalacturonic acid (5, 10, 15 and 20 µg). With this calibration curve the uronic acid content in a sample of 20 µg of the product was determined. The obtained result was a content of 95% uronic acid with respect to available 6-hydroxymethyl groups, the with a yield of 95%.
For partial oxidation (about 6%), a glucan produced as described before (dry weight 7.5 g, 46.4 mmol) was resuspended in 50 ml water. Next, TEMPO (1% by weight compared to the polysaccharide (0.075 g, 0.5 mmol)) was added, resuspended in 20 min and cooled to 0°C. A solution of hypochlorite (2.8 ml, 15% solution, 5.9 mmol) was adjusted to pH 9.0 with 3M HCl and cooled down to 0°C. This solution was added to the suspension of the polysaccharide and TEMPO. Within 5 min the mixture became a solid gel.
***Description of the figures***
SEQ ID No. 2: The deduced amino acid sequence of glucosyltransferase A (GTFA) of *Lactobacillus reuteri.*
Figure 1: The strategy used for the isolation of the *gtfA* gene from *Lactobacillus reuteri* 121 chromosomal DNA.
Figure 2: The general principle of the construction of the recombinant plasmid with the gtf*A* gene. A PCR product containing the gtf*A* gene was digested with *Xba*I and *Pst*I and with *Pst*I and *Bam*HI*.* The *Xba*I/*Pst*I (depicted in white) was ligated into the multiple cloning site of pBluescriptIISK⁺ in the same direction relatively to the *lacZ* promotor, resulting in pBXP1500. The *Pst*I/*Bam*HI part (depicted in grey) was ligated into the multiple cloning site of pBluescriptIISK⁺ in the opposite direction relatively to the *lacZ* promotor, resulting in pBPB2700. pBXP1500 was used as a vector for subcloning the C-terminal part of the gtf*A*. pBPB2700 was digested with *Pst*I and *Sal*I and ligated into pBXP1500, also digested with *Pst*I and *Sal*I*.* The resulting plasmid, pBGTF, contained the entire gtf*A* in the same direction relative to the *lacZ* promotor. The ∃ sign indicates the restriction.
Figure 3: The nucleotide and deduced amino acid sequence of *gtfA* of *Lactobacillus reuteri.* The start codon is shown in bold. The putative ribosomal binding site (RBS) is also shown in bold. The nucleotides in italic and underlined upstream of the start codon indicate the -10 region (Pribnow box) and the -35 region. The inverted repeats (transcription termination) after the stop codon are underlined. The * sign indicates the stop codon.
Figure 4: Alignment of catalytic cores of alternansucrase (ASR) of *Leuconostoc mesenteroides* strain NRRL B-1355 dextransucrase (DSRS) of *Leuconostoc mesenteroides* strain NRRL B-512F, glucosyltransferase-D (GTFD) of *Streptococcus mutans* GS5, glucosyltransferase-A of *Lactobacillus reuteri* and amylosucrase (AS) of *Neisseria polysaccharea.* * indicates identical or conserved residues in all sequences); ---, gap in the sequence; **AA** amino acids which are conserved in all other glucosyltranferases but not in GTFA; ⇓, putative catalytic residues; •, putative calcium binding sites; ◆, putative residues stabilising the transition state; ∇, residues possibly playing a role in binding of acceptor molecules and in the transfer of the glucosyl residue; 0, putative chloride binding sites; -Ex-, localisation of β-strands; -Hx-, localisation of α-helices according to Mac Gregor *et al.* (1996) FEBS Lett. 378, 262-266. The numbering of the amino acids of the glucosyltransferase-A of *Lactobacillus reuteri* corresponds to the positions of these amino acids in the amino acid sequence 531-1781 of amino acid sequence SEQ ID No. 2, when the amino acid sequence 531-1781 is renumbered 1-1251.
Figure 5: Dendrogram of glucansucrases of lactic acid bacteria. The horizontal distances are a measure for the difference at the amino acid sequence level. 10% difference is indicated by the upper bar. Bootstrap values (in percentages) are given at the root of each branch.
Figure 6: 500-MHz ¹H-NMR spectra of the glucan produced by *Lactobacillus reuteri* GTFA (A) and by *E*. *coli* GTFA (B), recorded in D₂O at 80°C.

## Claims

1. A protein having a molecular weight of about 190 kDa and having glucosyltransferase activity, comprising an amino acid sequence exhibiting at least 50% amino acid identity, as determined by the BLAST algorithm, with the amino acid sequence of SEQ ID No. 2.

2. A protein according to claim 1 comprising an amino acid sequence, exhibiting at least 60%, preferably at least 70%, amino acid identity with the amino acid sequence of SEQ ID No. 2.

3. A protein having glucosyltransferase activity comprising a part of an amino acid sequence exhibiting at least 50% amino acid identity, as determined by the BLAST algorithm, with the amino acid sequence 531-1781 of SEQ ID No. 2, said part comprising at least 100 amino acids exhibiting at least 65% amino acid identity with the corresponding part of the amino acid sequence 972-1514 of SEQ ID No. 2 and/or said part comprising at least 100 amino acids exhibiting at least 60% amino acid identity with the corresponding part of the amino acid sequence 1515-1781 of SEQ ID No. 2.

4. A protein according to claim 3, exhibiting at least 60%, preferably at least 70%, amino acid identity with the amino acid sequence 531-1781 of SEQ ID No. 2.

5. A protein according to any one of claims 1-4, comprising an amino acid sequence of at least 200 amino acids exhibiting at least 55%, preferably at least 65%, amino acid identity with the corresponding part of the amino acid sequence 972-1514 of SEQ ID No. 2.

6. A protein according to any one of claims 1-5, comprising an amino acid sequence of at least 100 amino acids exhibiting at least 50%, preferably at least 60%, amino acid identity with the corresponding part of the amino acid sequence 1515-1781 of SEQ ID No. 2.

7. A protein according to any one of claims 1-6, comprising at least one of the amino acids Pro-1026, Ile-1029, Met-1034, Asn-1035, Ser-1136, Ala-1143, Ile-1170, Leu-1223, Ala-1413, Val-1418, Ala-1428, Leu-1442 of the amino acid sequence of SEQ ID No. 2.

8. A protein according to any one of the preceding claims which, in the presence of sucrose, produces a glucan having 38-48% 4-linked anhydroglucose units, 17-28% 6-linked anhydroglucose units, and 7-20% 4,6-linked anhydroglucose units.

9. A protein according to any one of the preceding claims which is a recombinant protein.

10. A nucleotide sequence encoding a protein according to any one of the preceding claims.

11. A nucleic acid construct comprising the nucleic acid sequence of claim 10, operationally linked to an expression-regulating nucleic acid sequence.

12. A recombinant host cell containing one or more copies of the nucleic acid construct according to claim 11.

13. A process of producing a protein of interest, such as a glucosyltransferase, comprising culturing a host cell according to claim 12 in a culture medium, and recovering the protein from the culture medium or the cell free extract.

14. A process of producing an oligosaccharide or polysaccharide of interest, using an isolated protein according to any one of claims 1-9, or a host cell according to claim 12.


SEQUENCE LISTING

<110> Nederlandse Organisatie voor TNO

<120> Novel glucosyltransferase

<130> Novel glucosyltransferase

<140>
<141>

<150> 00201871.1
<151> 2000-05-25

<160> 9

<170> PatentIn Ver. 2.1

<210> 1
<211> 6026
<212> DNA
<213> Lactobacillus reuteri

<220>
<221> RBS
<222> (148)..(155)

<220>
<221> -10_signal
<222> (113)..(118)

<220>
<221> -35_signal
<222> (75)..(80)

<220>
<221> CDS
<222> (161)..(5506)

<400> 1
atacatattt tgggcttctt tttttgttta aaactgtaaa tttgaatttt atttgaaaaa 60

attttggcta gaatttgaaa attccctttg aaaaaataaa acatcatagt attataatac 120

cgataatcaa attgtttatt ttgatatgaa ggagattaaa atg gaa ata aag aaa 175
Met Glu Ile Lys Lys
1               5

cat ttt aag ttg tac aaa agc ggc aaa caa tgg gta aca gca gca gtg 223
His Phe Lys Leu Tyr Lys Ser Gly Lys Gln Trp Val Thr Ala Ala Val
                10                  15                  20

gct acc gtt gcc gtt tca act gcg ctt ctt tac gga gga gtt gcg cat 271
Ala Thr Val Ala Val Ser Thr Ala Leu Leu Tyr Gly Gly Val Ala His
            25                  30                  35

gct gac caa caa gtt cag caa gct tcc acg act caa gac caa act tct 319

Ala Asp Gln Gln Val Gln Gln Ala Ser Thr Thr Gln Asp Gln Thr Ser
        40                  45                  50

acc gta aat aat gat act gat aaa aca gta gct tta gat act aat act     367
Thr Val Asn Asn Asp Thr Asp Lys Thr Val Ala Leu Asp Thr Asn Thr
    55                  60                  65

gac cag tca gct caa aca act gat aaa aaa caa gta gta tca aat act     415
Asp Gln Ser Ala Gln Thr Thr Asp Lys Lys Gln Val Val Ser Asn Thr
70                  75                  80                  85

aac caa agc aaa act gat gac act tca aca gct gat aag aat tct act     463
Asn Gln Ser Lys Thr Asp Asp Thr Ser Thr Ala Asp Lys Asn Ser Thr
                90                  95                  100

tct aca cct gtt tct gtt ttg cca tct aat aat act gaa aaa caa gct     511
Ser Thr Pro Val Ser Val Leu Pro Ser Asn Asn Thr Glu Lys Gln Ala
            105                 110                 115

aaa aat tat aat gag caa gac aaa gga aac tat ggg aat att gat act     559
Lys Asn Tyr Asn Glu Gln Asp Lys Gly Asn Tyr Gly Asn Ile Asp Thr
        120                 125                 130

gct tac ttt agc aat aat caa ttg cat gtt tca gga tgg aat gca acg     607
Ala Tyr Phe Ser Asn Asn Gln Leu His Val Ser Gly Trp Asn Ala Thr
    135                 140                 145

aac gca tct caa gga aca aac agt cga caa atc att gtg cgt gat atc     655
Asn Ala Ser Gln Gly Thr Asn Ser Arg Gln Ile Ile Val Arg Asp Ile
150                 155                 160                 165

aca acc aat aat gaa tta ggt cgc act gat gta aca aac aat gtt gca     703
Thr Thr Asn Asn Glu Leu Gly Arg Thr Asp Val Thr Asn Asn Val Ala
                170                 175                 180

cgc cca gac gtt aag aat gtt cat aat gtt tat aac gct gat aat tct     751
Arg Pro Asp Val Lys Asn Val His Asn Val Tyr Asn Ala Asp Asn Ser
            185                 190                 195

gga ttt gat gtt aat gtc aat att gac ttt agc aag atg aaa gat tat     799
Gly Phe Asp Val Asn Val Asn Ile Asp Phe Ser Lys Met Lys Asp Tyr
        200                 205                 210

cgg gat tca att gaa att gtt agt cga tac agt gga aac ggt aaa tct     847
Arg Asp Ser Ile Glu Ile Val Ser Arg Tyr Ser Gly Asn Gly Lys Ser
    215                 220                 225

gtt gac tgg tgg tcc caa ccg atc act ttt gac aaa aac aac tat gct     895
Val Asp Trp Trp Ser Gln Pro Ile Thr Phe Asp Lys Asn Asn Tyr Ala
230                 235                 240                 245

tat ctt gat aca ttt gaa gtg aaa aat ggc gaa tta cat gca acc gga     943
Tyr Leu Asp Thr Phe Glu Val Lys Asn Gly Glu Leu His Ala Thr Gly
                250                 255                 260

tgg aat gct act aat agt gcg att aac tat aat cac cat ttt gtg att     991
Trp Asn Ala Thr Asn Ser Ala Ile Asn Tyr Asn His His Phe Val Ile

            265                 270                 275

ttg ttt gat caa acg aat ggt aaa gaa gta gca cga caa gaa gtt cgt 1039
Leu Phe Asp Gln Thr Asn Gly Lys Glu Val Ala Arg Gln Glu Val Arg
        280                 285                 290

gaa ggt caa tca cgc cca gat gtt gct aag gta tat cca caa gta gtt 1087
Glu Gly Gln Ser Arg Pro Asp Val Ala Lys Val Tyr Pro Gln Val Val
    295                 300                 305

ggt gct gcc aac tca ggc ttt aat gtg aca ttt aat atc agt gat tta 1135
Gly Ala Ala Asn Ser Gly Phe Asn Val Thr Phe Asn Ile Ser Asp Leu
310                 315                 320                 325

gat tat act cac cag tac caa gtt ctt agt cgt tac agc aat tct gat 1183
Asp Tyr Thr His Gln Tyr Gln Val Leu Ser Arg Tyr Ser Asn Ser Asp
                330                 335                 340

aat ggc gaa ggt gat aac gtt acc tac tgg ttt aat cca caa tcc att 1231
Asn Gly Glu Gly Asp Asn Val Thr Tyr Trp Phe Asn Pro Gln Ser Ile
            345                 350                 355

gct cct gct aat caa agt aac cag ggt tat cta gac tca ttt gat att 1279
Ala Pro Ala Asn Gln Ser Asn Gln Gly Tyr Leu Asp Ser Phe Asp Ile
        360                 365                 370

agt aaa aat ggt gaa gta aca gta act gga tgg aac gct act gac ttg 1327
Ser Lys Asn Gly Glu Val Thr Val Thr Gly Trp Asn Ala Thr Asp Leu
    375                 380                 385

tca gaa tta caa aac aac cat tat gtg att cta ttt gat cag aca gca 1375
Ser Glu Leu Gln Asn Asn His Tyr Val Ile Leu Phe Asp Gln Thr Ala
390                 395                 400                 405

ggc aaa caa gtt gca tct gct aaa gct gat tta att tca cgt cca gat 1423
Gly Lys Gln Val Ala Ser Ala Lys Ala Asp Leu Ile Ser Arg Pro Asp
                410                 415                 420

gtt gct aaa gct tat cca aca gta aaa aca gct aca aat tct ggc ttc 1471
Val Ala Lys Ala Tyr Pro Thr Val Lys Thr Ala Thr Asn Ser Gly Phe
            425                 430                 435

aag gta aca ttt aag gtt aat aac tta caa ccg ggt cac caa tac agc 1519
Lys Val Thr Phe Lys Val Asn Asn Leu Gln Pro Gly His Gln Tyr Ser
        440                 445                 450

gtt gta agt cgt ttc tct gcc gat gaa aat ggt aat ggt aat gat aag 1567
Val Val Ser Arg Phe Ser Ala Asp Glu Asn Gly Asn Gly Asn Asp Lys
    455                 460                 465

cgc cat aca gat tac tgg ttt agt cca gta ata tta aac cag act gct 1615
Arg His Thr Asp Tyr Trp Phe Ser Pro Val Ile Leu Asn Gln Thr Ala
470                 475                 480                 485

tca aac att gat act att aca atg aca tct aat ggt tta cat att gca 1663
Ser Asn Ile Asp Thr Ile Thr Met Thr Ser Asn Gly Leu His Ile Ala
                490                 495                 500

ggt tgg atg gca agt gat aac tca att aat gaa aca act cca tac gct    1711
Gly Trp Met Ala Ser Asp Asn Ser Ile Asn Glu Thr Thr Pro Tyr Ala
            505                 510                 515

att atc ctc aat aat gga aaa gaa gtt act cgt caa aag atg agc tta    1759
Ile Ile Leu Asn Asn Gly Lys Glu Val Thr Arg Gln Lys Met Ser Leu
        520                 525                 530

acc gcc cgt cca gat gta gca gca gta tat cct tca ctt tat aat agt    1807
Thr Ala Arg Pro Asp Val Ala Ala Val Tyr Pro Ser Leu Tyr Asn Ser
    535                 540                 545

gct gtt agt ggt ttt gac act act att aaa ttg act aat gat caa tat    1855
Ala Val Ser Gly Phe Asp Thr Thr Ile Lys Leu Thr Asn Asp Gln Tyr
550                 555                 560                 565

caa gcg ctt aat ggc caa tta caa gta ttg tta cgt ttt tct aaa gct    1903
Gln Ala Leu Asn Gly Gln Leu Gln Val Leu Leu Arg Phe Ser Lys Ala
                570                 575                 580

gct gat ggt aat cca agt ggt gat aat act gta act gat caa ttt agt    1951
Ala Asp Gly Asn Pro Ser Gly Asp Asn Thr Val Thr Asp Gln Phe Ser
            585                 590                 595

aaa aat tat gca act act ggt gga aac ttt gac tat gta aaa gta aat    1999
Lys Asn Tyr Ala Thr Thr Gly Gly Asn Phe Asp Tyr Val Lys Val Asn
        600                 605                 610

ggt aat caa gtt gaa ttt agt ggt tgg cac gca act aac caa tca aat    2047
Gly Asn Gln Val Glu Phe Ser Gly Trp His Ala Thr Asn Gln Ser Asn
    615                 620                 625

gat aaa gat tca caa tgg att att gtt tta gtt aat ggt aag gaa gta    2095
Asp Lys Asp Ser Gln Trp Ile Ile Val Leu Val Asn Gly Lys Glu Val
630                 635                 640                 645

aag cgt caa tta gtt aat gat act aaa gag gga gct gct ggc ttc aac    2143
Lys Arg Gln Leu Val Asn Asp Thr Lys Glu Gly Ala Ala Gly Phe Asn
                650                 655                 660

cga aac gat gtc tac aaa gta aat cca gct att gaa aac agt tct atg    2191
Arg Asn Asp Val Tyr Lys Val Asn Pro Ala Ile Glu Asn Ser Ser Met
            665                 670                 675

tct gga ttc caa ggc att att act tta cct gtg aca gtt aaa aac gaa    2239
Ser Gly Phe Gln Gly Ile Ile Thr Leu Pro Val Thr Val Lys Asn Glu
        680                 685                 690

aat gtc caa ctt gtt cat cgg ttt agt aac gat gtg aag act ggt gaa    2287
Asn Val Gln Leu Val His Arg Phe Ser Asn Asp Val Lys Thr Gly Glu
    695                 700                 705

ggt aac tat gtt gat ttc tgg tca gag cta atg cct gtt aag gat agc    2335
Gly Asn Tyr Val Asp Phe Trp Ser Glu Leu Met Pro Val Lys Asp Ser
710                 715                 720                 725

ttc caa aag ggg aat ggc cca ctt aag caa ttt ggc tta caa act att     2383
Phe Gln Lys Gly Asn Gly Pro Leu Lys Gln Phe Gly Leu Gln Thr Ile
                730                 735                 740

aac ggt caa caa tat tat att gac cca aca act ggt caa cca cgt aag     2431
Asn Gly Gln Gln Tyr Tyr Ile Asp Pro Thr Thr Gly Gln Pro Arg Lys
            745                 750                 755

aat ttc tta tta caa agt gga aat aat tgg att tac ttt gat agt gat     2479
Asn Phe Leu Leu Gln Ser Gly Asn Asn Trp Ile Tyr Phe Asp Ser Asp
        760                 765                 770

act ggt gtg ggt act aat gca ctt gaa tta caa ttt gca aag gga act     2527
Thr Gly Val Gly Thr Asn Ala Leu Glu Leu Gln Phe Ala Lys Gly Thr
    775                 780                 785

gtt tca tct aat gaa caa tac cgt aac ggt aat gca gct tac agt tat     2575
Val Ser Ser Asn Glu Gln Tyr Arg Asn Gly Asn Ala Ala Tyr Ser Tyr
790                 795                 800                 805

gat gac aag agt atc gaa aat gta aat ggt tac tta aca gca gat aca     2623
Asp Asp Lys Ser Ile Glu Asn Val Asn Gly Tyr Leu Thr Ala Asp Thr
                810                 815                 820

tgg tac cgt cca aaa cag atc tta aag gat gga act acc tgg act gac     2671
Trp Tyr Arg Pro Lys Gln Ile Leu Lys Asp Gly Thr Thr Trp Thr Asp
            825                 830                 835

tca aaa gaa aca gat atg cga cca atc ttg atg gta tgg tgg cct aat     2719
Ser Lys Glu Thr Asp Met Arg Pro Ile Leu Met Val Trp Trp Pro Asn
        840                 845                 850

act ctt acc caa gca tac tac ctt aat tac atg aaa caa cat ggt aat     2767
Thr Leu Thr Gln Ala Tyr Tyr Leu Asn Tyr Met Lys Gln His Gly Asn
    855                 860                 865

tta tta cca tct gct tta cca ttc ttt aat gcg gat gct gat cct gca     2815
Leu Leu Pro Ser Ala Leu Pro Phe Phe Asn Ala Asp Ala Asp Pro Ala
870                 875                 880                 885

gaa tta aat cat tat tcc gaa att gtg caa caa aat att gaa aaa cga     2863
Glu Leu Asn His Tyr Ser Glu Ile Val Gln Gln Asn Ile Glu Lys Arg
                890                 895                 900

att agt gaa acc gga aat act gat tgg tta cgt act tta atg cac gat     2911
Ile Ser Glu Thr Gly Asn Thr Asp Trp Leu Arg Thr Leu Met His Asp
            905                 910                 915

ttt gtt act aac aat ccg atg tgg aat aag gat agt gaa aat gtt aac     2959
Phe Val Thr Asn Asn Pro Met Trp Asn Lys Asp Ser Glu Asn Val Asn
        920                 925                 930

ttt agt ggt att caa ttc caa ggc gga ttc tta aag tat gaa aac tca     3007
Phe Ser Gly Ile Gln Phe Gln Gly Gly Phe Leu Lys Tyr Glu Asn Ser
    935                 940                 945

gat tta acg cct tat gct aac tct gat tat cgc tta ctt ggt cgg atg     3055

Asp Leu Thr Pro Tyr Ala Asn Ser Asp Tyr Arg Leu Leu Gly Arg Met
950                 955                 960                 965

cca atc aat att aag gat caa aca tat cgg gga caa gaa ttc cta ctt     3103
Pro Ile Asn Ile Lys Asp Gln Thr Tyr Arg Gly Gln Glu Phe Leu Leu
                970                 975                 980

gct aac gat att gat aac tct aat cct gtt gtt caa gca gaa caa tta     3151
Ala Asn Asp Ile Asp Asn Ser Asn Pro Val Val Gln Ala Glu Gln Leu
            985                 990                 995

aac tgg tta tac tat ctc ttg aac ttt gga acg atc aca gct aat aat     3199
Asn Trp Leu Tyr Tyr Leu Leu Asn Phe Gly Thr Ile Thr Ala Asn Asn
        1000                1005                1010

gat caa gct aat ttt gat tct gta cgg gta gat gca ccg gat aat att     3247
Asp Gln Ala Asn Phe Asp Ser Val Arg Val Asp Ala Pro Asp Asn Ile
    1015                1020                1025

gat gcc gat ctt atg aat atc gct cag gac tac ttt aat gct gca tat     3295
Asp Ala Asp Leu Met Asn Ile Ala Gln Asp Tyr Phe Asn Ala Ala Tyr
1030                1035                1040                1045

ggt atg gac tca gat gct gtc tca aat aag cat att aat att ctt gaa     3343
Gly Met Asp Ser Asp Ala Val Ser Asn Lys His Ile Asn Ile Leu Glu
                1050                1055                1060

gac tgg aat cat gct gat ccg gaa tac ttt aat aag atc gga aat cca     3391
Asp Trp Asn His Ala Asp Pro Glu Tyr Phe Asn Lys Ile Gly Asn Pro
            1065                1070                1075

caa ttg aca atg gat gat act att aag aat tcc ctg aat cat ggg ctt     3439
Gln Leu Thr Met Asp Asp Thr Ile Lys Asn Ser Leu Asn His Gly Leu
        1080                1085                1090

tca gat gca act aat cgt tgg gga tta gat gca att gtt cat cag tca     3487
Ser Asp Ala Thr Asn Arg Trp Gly Leu Asp Ala Ile Val His Gln Ser
    1095                1100                1105

tta gct gat cgt gaa aat aat tcc acg gaa aat gtt gta att cct aat     3535
Leu Ala Asp Arg Glu Asn Asn Ser Thr Glu Asn Val Val Ile Pro Asn
1110                1115                1120                1125

tac agt ttc gtt cgg gct cac gat aat aat tct caa gat caa att caa     3583
Tyr Ser Phe Val Arg Ala His Asp Asn Asn Ser Gln Asp Gln Ile Gln
                1130                1135                1140

aat gct att cgt gat gta aca ggc aaa gat tac cat act ttc act ttt     3631
Asn Ala Ile Arg Asp Val Thr Gly Lys Asp Tyr His Thr Phe Thr Phe
            1145                1150                1155

gaa gat gag caa aag ggt att gat gcg tac att caa gat caa aat tca     3679
Glu Asp Glu Gln Lys Gly Ile Asp Ala Tyr Ile Gln Asp Gln Asn Ser
        1160                1165                1170

aca gtg aag aaa tat aac ctt tat aat att ccg gct tca tac gca att     3727
Thr Val Lys Lys Tyr Asn Leu Tyr Asn Ile Pro Ala Ser Tyr Ala Ile

1175                1180                1185

ctt tta act aac aag gat aca att cca cgt gta tac tat ggt gac ttg    3775
Leu Leu Thr Asn Lys Asp Thr Ile Pro Arg Val Tyr Tyr Gly Asp Leu
1190                1195                1200                1205

tat act gat ggt ggc caa tac atg gaa cat caa aca cgt tac tat gat    3823
Tyr Thr Asp Gly Gly Gln Tyr Met Glu His Gln Thr Arg Tyr Tyr Asp
                1210                1215                1220

act tta acg aac ctg ctt aaa tca cga gtt aag tat gtt gcc ggt ggc    3871
Thr Leu Thr Asn Leu Leu Lys Ser Arg Val Lys Tyr Val Ala Gly Gly
            1225                1230                1235

caa tca atg caa aca atg agc gtt ggc ggc aat aat aac att tta act    3919
Gln Ser Met Gln Thr Met Ser Val Gly Gly Asn Asn Asn Ile Leu Thr
        1240                1245                1250

agt gtt cgt tat ggt aaa ggt gcg atg aca gct act gat act ggt act    3967
Ser Val Arg Tyr Gly Lys Gly Ala Met Thr Ala Thr Asp Thr Gly Thr
    1255                1260                1265

gat gaa acc aga aca caa ggt att ggg gtt gtt gta agt aat acg cca    4015
Asp Glu Thr Arg Thr Gln Gly Ile Gly Val Val Val Ser Asn Thr Pro
1270                1275                1280                1285

aat cta aag cta ggt gtc aac gat aaa gta gtt ctt cat atg gga gct    4063
Asn Leu Lys Leu Gly Val Asn Asp Lys Val Val Leu His Met Gly Ala
                1290                1295                1300

gcg cac aag aac caa caa tat cgg gca gcc gtg ttg acg aca act gat    4111
Ala His Lys Asn Gln Gln Tyr Arg Ala Ala Val Leu Thr Thr Thr Asp
            1305                1310                1315

gga gtc att aat tat act tct gat caa ggg gca ccg gtt gca atg act    4159
Gly Val Ile Asn Tyr Thr Ser Asp Gln Gly Ala Pro Val Ala Met Thr
        1320                1325                1330

gac gag aac ggt gat cta tac tta tct agt cat aac cta gtt gtt aat    4207
Asp Glu Asn Gly Asp Leu Tyr Leu Ser Ser His Asn Leu Val Val Asn
    1335                1340                1345

ggt aaa gaa gaa gca gat aca gct gtt caa ggt tat gct aac cct gat    4255
Gly Lys Glu Glu Ala Asp Thr Ala Val Gln Gly Tyr Ala Asn Pro Asp
1350                1355                1360                1365

gtt tca gga tat ctt gct gta tgg gta cca gtt gga gca agt gat aac    4303
Val Ser Gly Tyr Leu Ala Val Trp Val Pro Val Gly Ala Ser Asp Asn
                1370                1375                1380

caa gat gct cga act gct cca tct act gaa aag aat agt ggt aac tct    4351
Gln Asp Ala Arg Thr Ala Pro Ser Thr Glu Lys Asn Ser Gly Asn Ser
            1385                1390                1395

gca tac aga aca aat gct gct ttt gat tca aat gtt att ttt gaa gcc    4399
Ala Tyr Arg Thr Asn Ala Ala Phe Asp Ser Asn Val Ile Phe Glu Ala
        1400                1405                1410

ttt tct aac ttt gtc tat aca cca aca aag gaa agt gaa cgt gct aat     4447
Phe Ser Asn Phe Val Tyr Thr Pro Thr Lys Glu Ser Glu Arg Ala Asn
    1415                1420                1425

gtt cga att gcc caa aat gct gat ttc ttt gct tca tta ggt ttt act     4495
Val Arg Ile Ala Gln Asn Ala Asp Phe Phe Ala Ser Leu Gly Phe Thr
1430                1435                1440                1445

tct ttc gag atg gcg cca caa tat aat tca agt aaa gat cgc aca ttc     4543
Ser Phe Glu Met Ala Pro Gln Tyr Asn Ser Ser Lys Asp Arg Thr Phe
                1450                1455                1460

cta gat tca aca att gat aac gga tat gcg ttt act gat cgt tat gat     4591
Leu Asp Ser Thr Ile Asp Asn Gly Tyr Ala Phe Thr Asp Arg Tyr Asp
            1465                1470                1475

ctt gga atg agt gag cct aat aag tac gga aca gat gaa gat cta cgt     4639
Leu Gly Met Ser Glu Pro Asn Lys Tyr Gly Thr Asp Glu Asp Leu Arg
        1480                1485                1490

aat gcc att caa gcg ctc cat aaa gct ggc tta caa gta atg gcg gat     4687
Asn Ala Ile Gln Ala Leu His Lys Ala Gly Leu Gln Val Met Ala Asp
    1495                1500                1505

tgg gtt cct gac caa atc tat aac ctt cct gga aaa gaa gtt gct aca     4735
Trp Val Pro Asp Gln Ile Tyr Asn Leu Pro Gly Lys Glu Val Ala Thr
1510                1515                1520                1525

gtc act cga gta gat gat cgt ggt aat gta tgg aaa gat gct atc att     4783
Val Thr Arg Val Asp Asp Arg Gly Asn Val Trp Lys Asp Ala Ile Ile
                1530                1535                1540

aat aat aat ctg tat gtt gtt aat act att ggt ggt ggc gaa tac cag     4831
Asn Asn Asn Leu Tyr Val Val Asn Thr Ile Gly Gly Gly Glu Tyr Gln
            1545                1550                1555

aag aag tat ggt gga gca ttc ctc gat aag tta caa aaa ctt tat cct     4879
Lys Lys Tyr Gly Gly Ala Phe Leu Asp Lys Leu Gln Lys Leu Tyr Pro
        1560                1565                1570

gaa atc ttc aca aag aag caa gtt tca act ggt gtt gct att gat cct     4927
Glu Ile Phe Thr Lys Lys Gln Val Ser Thr Gly Val Ala Ile Asp Pro
    1575                1580                1585

tca caa aag ata act gaa tgg tca gca aaa tac ttt aat gga aca aac     4975
Ser Gln Lys Ile Thr Glu Trp Ser Ala Lys Tyr Phe Asn Gly Thr Asn
1590                1595                1600                1605

att ctc cat cgt ggt tct ggt tat gta cta aaa gct gat ggt ggt caa     5023
Ile Leu His Arg Gly Ser Gly Tyr Val Leu Lys Ala Asp Gly Gly Gln
                1610                1615                1620

tac tac aac tta ggt act act aca aag caa ttc ttg cca att caa tta     5071
Tyr Tyr Asn Leu Gly Thr Thr Thr Lys Gln Phe Leu Pro Ile Gln Leu
            1625                1630                1635

act ggt gaa aag aaa caa gga aat gaa ggc ttt gtt aag ggt aat gat    5119
Thr Gly Glu Lys Lys Gln Gly Asn Glu Gly Phe Val Lys Gly Asn Asp
        1640                1645                1650

gga aat tac tac ttc tat gac tta gca ggt aat atg gtt aag aat acc    5167
Gly Asn Tyr Tyr Phe Tyr Asp Leu Ala Gly Asn Met Val Lys Asn Thr
    1655                1660                1665

ttt att gaa gat agt gtt ggc aac tgg tac ttc ttt gac caa gat ggt    5215
Phe Ile Glu Asp Ser Val Gly Asn Trp Tyr Phe Phe Asp Gln Asp Gly
1670                1675                1680                1685

aag atg gtt gaa aat aaa cat ttc gtt gat gtt gat tct tat ggt gaa    5263
Lys Met Val Glu Asn Lys His Phe Val Asp Val Asp Ser Tyr Gly Glu
                1690                1695                1700

aaa ggt act tac ttc ttc ttg aag aat ggt gta tca ttc cgt ggg gga    5311
Lys Gly Thr Tyr Phe Phe Leu Lys Asn Gly Val Ser Phe Arg Gly Gly
            1705                1710                1715

tta gtg caa act gac aat ggt act tat tac ttt gat aat tat gga aag    5359
Leu Val Gln Thr Asp Asn Gly Thr Tyr Tyr Phe Asp Asn Tyr Gly Lys
        1720                1725                1730

atg gta cgt aat caa act att aat gca ggt gcc atg att tat acc tta    5407
Met Val Arg Asn Gln Thr Ile Asn Ala Gly Ala Met Ile Tyr Thr Leu
    1735                1740                1745

gat gaa aac ggt aag ctt ata aag gct agt tat aat tca gat gcc gaa    5455
Asp Glu Asn Gly Lys Leu Ile Lys Ala Ser Tyr Asn Ser Asp Ala Glu
1750                1755                1760                1765

tat cca act tca act gat gtt ggt aag atg ctt gat caa aat aaa cta    5503
Tyr Pro Thr Ser Thr Asp Val Gly Lys Met Leu Asp Gln Asn Lys Leu
                1770                1775                1780

taa atggaaataa ttagctgatt tccgtttctt agaatcgaaa gatttaataa        5556

ctggggttaa aacggcccta caaaatctga tattgatata gagatattat ttcctatatc 5616

aatatcagat ttttgctttt tataaaattg attgtgacta ataagaatcc ggaagataac 5676

gttgttgtta tatcagtgga tttaagcaac atgaattaat tgaagatgac ggcaatgatt 5736

aaaagtcggt ctgatgatta ttgatgtatt actagtattt ggtttttatc atttatattt 5796

ttactgttat tggtgtcata tattccacaa taacagtaaa ggtatatatg ctagtttatt 5856

ttttaagtaa ttataatatt ctgattataa tttggaaata ttcgctttta gcaaaaaggt 5916

agtaaacaga tcagaatcgt cattctgctt ttctactact aaaagtctgt tttaaattct 5976

aaactaaaat aggctaaaca ctgatgttta tcatttatat ttttactgtt            6026

<210> 2

<211> 1781
<212> PRT
<213> Lactobacillus reuteri

<400> 2
Met Glu Ile Lys Lys His Phe Lys Leu Tyr Lys Ser Gly Lys Gln Trp
1               5                   10                  15

Val Thr Ala Ala Val Ala Thr Val Ala Val Ser Thr Ala Leu Leu Tyr
            20                  25                  30

Gly Gly Val Ala His Ala Asp Gln Gln Val Gln Gln Ala Ser Thr Thr
        35                  40                  45

Gln Asp Gln Thr Ser Thr Val Asn Asn Asp Thr Asp Lys Thr Val Ala
    50                  55                  60

Leu Asp Thr Asn Thr Asp Gln Ser Ala Gln Thr Thr Asp Lys Lys Gln
65                  70                  75                  80

Val Val Ser Asn Thr Asn Gln Ser Lys Thr Asp Asp Thr Ser Thr Ala
                85                  90                  95

Asp Lys Asn Ser Thr Ser Thr Pro Val Ser Val Leu Pro Ser Asn Asn
            100                 105                 110

Thr Glu Lys Gln Ala Lys Asn Tyr Asn Glu Gln Asp Lys Gly Asn Tyr
        115                 120                 125

Gly Asn Ile Asp Thr Ala Tyr Phe Ser Asn Asn Gln Leu His Val Ser
    130                 135                 140

Gly Trp Asn Ala Thr Asn Ala Ser Gln Gly Thr Asn Ser Arg Gln Ile
145                 150                 155                 160

Ile Val Arg Asp Ile Thr Thr Asn Asn Glu Leu Gly Arg Thr Asp Val
                165                 170                 175

Thr Asn Asn Val Ala Arg Pro Asp Val Lys Asn Val His Asn Val Tyr
            180                 185                 190

Asn Ala Asp Asn Ser Gly Phe Asp Val Asn Val Asn Ile Asp Phe Ser
        195                 200                 205

Lys Met Lys Asp Tyr Arg Asp Ser Ile Glu Ile Val Ser Arg Tyr Ser
    210                 215                 220

Gly Asn Gly Lys Ser Val Asp Trp Trp Ser Gln Pro Ile Thr Phe Asp
225                 230                 235                 240

Lys Asn Asn Tyr Ala Tyr Leu Asp Thr Phe Glu Val Lys Asn Gly Glu
                245                 250                 255

Leu His Ala Thr Gly Trp Asn Ala Thr Asn Ser Ala Ile Asn Tyr Asn
            260                 265                 270

His His Phe Val Ile Leu Phe Asp Gln Thr Asn Gly Lys Glu Val Ala

275                 280                 285

Arg Gln Glu Val Arg Glu Gly Gln Ser Arg Pro Asp Val Ala Lys Val
    290                 295                 300

Tyr Pro Gln Val Val Gly Ala Ala Asn Ser Gly Phe Asn Val Thr Phe
305                 310                 315                 320

Asn Ile Ser Asp Leu Asp Tyr Thr His Gln Tyr Gln Val Leu Ser Arg
                325                 330                 335

Tyr Ser Asn Ser Asp Asn Gly Glu Gly Asp Asn Val Thr Tyr Trp Phe
            340                 345                 350

Asn Pro Gln Ser Ile Ala Pro Ala Asn Gln Ser Asn Gln Gly Tyr Leu
        355                 360                 365

Asp Ser Phe Asp Ile Ser Lys Asn Gly Glu Val Thr Val Thr Gly Trp
    370                 375                 380

Asn Ala Thr Asp Leu Ser Glu Leu Gln Asn Asn His Tyr Val Ile Leu
385                 390                 395                 400

Phe Asp Gln Thr Ala Gly Lys Gln Val Ala Ser Ala Lys Ala Asp Leu
                405                 410                 415

Ile Ser Arg Pro Asp Val Ala Lys Ala Tyr Pro Thr Val Lys Thr Ala
            420                 425                 430

Thr Asn Ser Gly Phe Lys Val Thr Phe Lys Val Asn Asn Leu Gln Pro
        435                 440                 445

Gly His Gln Tyr Ser Val Val Ser Arg Phe Ser Ala Asp Glu Asn Gly
    450                 455                 460

Asn Gly Asn Asp Lys Arg His Thr Asp Tyr Trp Phe Ser Pro Val Ile
465                 470                 475                 480

Leu Asn Gln Thr Ala Ser Asn Ile Asp Thr Ile Thr Met Thr Ser Asn
                485                 490                 495

Gly Leu His Ile Ala Gly Trp Met Ala Ser Asp Asn Ser Ile Asn Glu
            500                 505                 510

Thr Thr Pro Tyr Ala Ile Ile Leu Asn Asn Gly Lys Glu Val Thr Arg
        515                 520                 525

Gln Lys Met Ser Leu Thr Ala Arg Pro Asp Val Ala Ala Val Tyr Pro
    530                 535                 540

Ser Leu Tyr Asn Ser Ala Val Ser Gly Phe Asp Thr Thr Ile Lys Leu
545                 550                 555                 560

Thr Asn Asp Gln Tyr Gln Ala Leu Asn Gly Gln Leu Gln Val Leu Leu
                565                 570                 575

Arg Phe Ser Lys Ala Ala Asp Gly Asn Pro Ser Gly Asp Asn Thr Val

580                 585                 590

Thr Asp Gln Phe Ser Lys Asn Tyr Ala Thr Thr Gly Gly Asn Phe Asp
        595                 600                 605

Tyr Val Lys Val Asn Gly Asn Gln Val Glu Phe Ser Gly Trp His Ala
    610                 615                 620

Thr Asn Gln Ser Asn Asp Lys Asp Ser Gln Trp Ile Ile Val Leu Val
625                 630                 635                 640

Asn Gly Lys Glu Val Lys Arg Gln Leu Val Asn Asp Thr Lys Glu Gly
                645                 650                 655

Ala Ala Gly Phe Asn Arg Asn Asp Val Tyr Lys Val Asn Pro Ala Ile
            660                 665                 670

Glu Asn Ser Ser Met Ser Gly Phe Gln Gly Ile Ile Thr Leu Pro Val
        675                 680                 685

Thr Val Lys Asn Glu Asn Val Gln Leu Val His Arg Phe Ser Asn Asp
    690                 695                 700

Val Lys Thr Gly Glu Gly Asn Tyr Val Asp Phe Trp Ser Glu Leu Met
705                 710                 715                 720

Pro Val Lys Asp Ser Phe Gln Lys Gly Asn Gly Pro Leu Lys Gln Phe
                725                 730                 735

Gly Leu Gln Thr Ile Asn Gly Gln Gln Tyr Tyr Ile Asp Pro Thr Thr
            740                 745                 750

Gly Gln Pro Arg Lys Asn Phe Leu Leu Gln Ser Gly Asn Asn Trp Ile
        755                 760                 765

Tyr Phe Asp Ser Asp Thr Gly Val Gly Thr Asn Ala Leu Glu Leu Gln
    770                 775                 780

Phe Ala Lys Gly Thr Val Ser Ser Asn Glu Gln Tyr Arg Asn Gly Asn
785                 790                 795                 800

Ala Ala Tyr Ser Tyr Asp Asp Lys Ser Ile Glu Asn Val Asn Gly Tyr
                805                 810                 815

Leu Thr Ala Asp Thr Trp Tyr Arg Pro Lys Gln Ile Leu Lys Asp Gly
            820                 825                 830

Thr Thr Trp Thr Asp Ser Lys Glu Thr Asp Met Arg Pro Ile Leu Met
        835                 840                 845

Val Trp Trp Pro Asn Thr Leu Thr Gln Ala Tyr Tyr Leu Asn Tyr Met
    850                 855                 860

Lys Gln His Gly Asn Leu Leu Pro Ser Ala Leu Pro Phe Phe Asn Ala
865                 870                 875                 880

Asp Ala Asp Pro Ala Glu Leu Asn His Tyr Ser Glu Ile Val Gln Gln

885                 890                 895

Asn Ile Glu Lys Arg Ile Ser Glu Thr Gly Asn Thr Asp Trp Leu Arg
            900                 905                 910

Thr Leu Met His Asp Phe Val Thr Asn Asn Pro Met Trp Asn Lys Asp
        915                 920                 925

Ser Glu Asn Val Asn Phe Ser Gly Ile Gln Phe Gln Gly Gly Phe Leu
    930                 935                 940

Lys Tyr Glu Asn Ser Asp Leu Thr Pro Tyr Ala Asn Ser Asp Tyr Arg
945                 950                 955                 960

Leu Leu Gly Arg Met Pro Ile Asn Ile Lys Asp Gln Thr Tyr Arg Gly
                965                 970                 975

Gln Glu Phe Leu Leu Ala Asn Asp Ile Asp Asn Ser Asn Pro Val Val
            980                 985                 990

Gln Ala Glu Gln Leu Asn Trp Leu Tyr Tyr Leu Leu Asn Phe Gly Thr
        995                 1000                1005

Ile Thr Ala Asn Asn Asp Gln Ala Asn Phe Asp Ser Val Arg Val Asp
   1010                1015                1020

Ala Pro Asp Asn Ile Asp Ala Asp Leu Met Asn Ile Ala Gln Asp Tyr
025                 1030                1035                1040

Phe Asn Ala Ala Tyr Gly Met Asp Ser Asp Ala Val Ser Asn Lys His
               1045                1050                1055

Ile Asn Ile Leu Glu Asp Trp Asn His Ala Asp Pro Glu Tyr Phe Asn
           1060                1065                1070

Lys Ile Gly Asn Pro Gln Leu Thr Met Asp Asp Thr Ile Lys Asn Ser
       1075                1080                1085

Leu Asn His Gly Leu Ser Asp Ala Thr Asn Arg Trp Gly Leu Asp Ala
   1090                1095                1100

Ile Val His Gln Ser Leu Ala Asp Arg Glu Asn Asn Ser Thr Glu Asn
105                 1110                1115                1120

Val Val Ile Pro Asn Tyr Ser Phe Val Arg Ala His Asp Asn Asn Ser
               1125                1130                1135

Gln Asp Gln Ile Gln Asn Ala Ile Arg Asp Val Thr Gly Lys Asp Tyr
           1140                1145                1150

His Thr Phe Thr Phe Glu Asp Glu Gln Lys Gly Ile Asp Ala Tyr Ile
       1155                1160                1165

Gln Asp Gln Asn Ser Thr Val Lys Lys Tyr Asn Leu Tyr Asn Ile Pro
   1170                1175                1180

Ala Ser Tyr Ala Ile Leu Leu Thr Asn Lys Asp Thr Ile Pro Arg Val

185                 1190                1195                1200

Tyr Tyr Gly Asp Leu Tyr Thr Asp Gly Gly Gln Tyr Met Glu His Gln
                1205                1210                1215

Thr Arg Tyr Tyr Asp Thr Leu Thr Asn Leu Leu Lys Ser Arg Val Lys
            1220                1225                1230

Tyr Val Ala Gly Gly Gln Ser Met Gln Thr Met Ser Val Gly Gly Asn
        1235                1240                1245

Asn Asn Ile Leu Thr Ser Val Arg Tyr Gly Lys Gly Ala Met Thr Ala
    1250                1255                1260

Thr Asp Thr Gly Thr Asp Glu Thr Arg Thr Gln Gly Ile Gly Val Val
265                 1270                1275                1280

Val Ser Asn Thr Pro Asn Leu Lys Leu Gly Val Asn Asp Lys Val Val
                1285                1290                1295

Leu His Met Gly Ala Ala His Lys Asn Gln Gln Tyr Arg Ala Ala Val
            1300                1305                1310

Leu Thr Thr Thr Asp Gly Val Ile Asn Tyr Thr Ser Asp Gln Gly Ala
        1315                1320                1325

Pro Val Ala Met Thr Asp Glu Asn Gly Asp Leu Tyr Leu Ser Ser His
    1330                1335                1340

Asn Leu Val Val Asn Gly Lys Glu Glu Ala Asp Thr Ala Val Gln Gly
345                 1350                1355                1360

Tyr Ala Asn Pro Asp Val Ser Gly Tyr Leu Ala Val Trp Val Pro Val
                1365                1370                1375

Gly Ala Ser Asp Asn Gln Asp Ala Arg Thr Ala Pro Ser Thr Glu Lys
            1380                1385                1390

Asn Ser Gly Asn Ser Ala Tyr Arg Thr Asn Ala Ala Phe Asp Ser Asn
        1395                1400                1405

Val Ile Phe Glu Ala Phe Ser Asn Phe Val Tyr Thr Pro Thr Lys Glu
    1410                1415                1420

Ser Glu Arg Ala Asn Val Arg Ile Ala Gln Asn Ala Asp Phe Phe Ala
425                 1430                1435                1440

Ser Leu Gly Phe Thr Ser Phe Glu Met Ala Pro Gln Tyr Asn Ser Ser
                1445                1450                1455

Lys Asp Arg Thr Phe Leu Asp Ser Thr Ile Asp Asn Gly Tyr Ala Phe
            1460                1465                1470

Thr Asp Arg Tyr Asp Leu Gly Met Ser Glu Pro Asn Lys Tyr Gly Thr
        1475                1480                1485

Asp Glu Asp Leu Arg Asn Ala Ile Gln Ala Leu His Lys Ala Gly Leu

1490                1495                1500

Gln Val Met Ala Asp Trp Val Pro Asp Gln Ile Tyr Asn Leu Pro Gly
505                 1510                1515                1520

Lys Glu Val Ala Thr Val Thr Arg Val Asp Asp Arg Gly Asn Val Trp
                1525                1530                1535

Lys Asp Ala Ile Ile Asn Asn Asn Leu Tyr Val Val Asn Thr Ile Gly
            1540                1545                1550

Gly Gly Glu Tyr Gln Lys Lys Tyr Gly Gly Ala Phe Leu Asp Lys Leu
        1555                1560                1565

Gln Lys Leu Tyr Pro Glu Ile Phe Thr Lys Lys Gln Val Ser Thr Gly
    1570                1575                1580

Val Ala Ile Asp Pro Ser Gln Lys Ile Thr Glu Trp Ser Ala Lys Tyr
585                 1590                1595                1600

Phe Asn Gly Thr Asn Ile Leu His Arg Gly Ser Gly Tyr Val Leu Lys
                1605                1610                1615

Ala Asp Gly Gly Gln Tyr Tyr Asn Leu Gly Thr Thr Thr Lys Gln Phe
            1620                1625                1630

Leu Pro Ile Gln Leu Thr Gly Glu Lys Lys Gln Gly Asn Glu Gly Phe
        1635                1640                1645

Val Lys Gly Asn Asp Gly Asn Tyr Tyr Phe Tyr Asp Leu Ala Gly Asn
    1650                1655                1660

Met Val Lys Asn Thr Phe Ile Glu Asp Ser Val Gly Asn Trp Tyr Phe
665                 1670                1675                1680

Phe Asp Gln Asp Gly Lys Met Val Glu Asn Lys His Phe Val Asp Val
                1685                1690                1695

Asp Ser Tyr Gly Glu Lys Gly Thr Tyr Phe Phe Leu Lys Asn Gly Val
            1700                1705                1710

Ser Phe Arg Gly Gly Leu Val Gln Thr Asp Asn Gly Thr Tyr Tyr Phe
        1715                1720                1725

Asp Asn Tyr Gly Lys Met Val Arg Asn Gln Thr Ile Asn Ala Gly Ala
    1730                1735                1740

Met Ile Tyr Thr Leu Asp Glu Asn Gly Lys Leu Ile Lys Ala Ser Tyr
745                 1750                1755                1760

Asn Ser Asp Ala Glu Tyr Pro Thr Ser Thr Asp Val Gly Lys Met Leu
                1765                1770                1775

Asp Gln Asn Lys Leu
            1780

<210> 3
<211> 13
<212> PRT
<213> Lactobacillus reuteri

<220>
<221> REPEAT
<222> (1)..(13)

<400> 3
Tyr Tyr Phe Tyr Asp Leu Ala Gly Asn Met Val Lys Asn
1               5                   10

<210> 4
<211> 13
<212> PRT
<213> Lactobacillus reuteri

<220>
<221> REPEAT
<222> (1)..(13)

<400> 4
Trp Tyr Phe Phe Asp Gln Asp Gly Lys Met Val Glu Asn
1               5                   10

<210> 5
<211> 13
<212> PRT
<213> Lactobacillus reuteri

<220>
<221> REPEAT
<222> (1)..(13)

<400> 5
Thr Tyr Tyr Phe Asp Asn Tyr Gly Lys Met Val Arg Asn
1               5                   10

<210> 6
<211> 26
<212> DNA
<213> Artificial Sequence

<220>
<223> Description of Artificial Sequence:Degenerate
      primer GTFpr1

<400> 6
gayaakwsna aksynrtngt nsargc                                          26

<210> 7
<211> 22
<212> DNA
<213> Artificial Sequence

<220>
<223> Description of Artificial Sequence:Degenerate
      primer GTFpr2

<400> 7
gnkcncanat ratrccnctr na                                                22


<210> 8
<211> 35
<212> DNA
<213> Artificial Sequence

<220>
<223> Description of Artificial Sequence:Degenerate
      primer GTFpr3

<400> 8
acaaccacca tggaattagg tcgcactgat gtaac                                  35


<210> 9
<211> 47
<212> DNA
<213> Artificial Sequence

<220>
<223> Description of Artificial Sequence:Degenerate
      primer GTFpr4

<400> 9
gccagctgga tccgtcgact agtttatttt tgatcaagca tcttacc                     47